Europäisches Patentamt

European Patent Office

Office européen des brevets

(19)

(11) Publication number: **0 095 350 B1**

## (12) EUROPEAN PATENT SPECIFICATION

(45) Date of publication of patent specification: **27.02.91**

(51) Int. Cl.⁵: **C12N 15/23, C12P 21/02, C12N 1/00, C12N 5/00, C07H 21/04, C07C 233/01, C12R 1/185, C12R 1/07**

(21) Application number: **83302915.0**

(22) Date of filing: **20.05.83**

The file contains technical information submitted after the application was filed and not included in this specification

(54) **A method of producing human gamma-interferon-like polipeptide.**

(30) Priority: **20.05.82 JP 86180/82**

(43) Date of publication of application:
**30.11.83 Bulletin 83/48**

(45) Publication of the grant of the patent:
**27.02.91 Bulletin 91/09**

(84) Designated Contracting States:
**AT BE CH DE FR GB IT LI LU NL SE**

(56) References cited:
**EP-A- 0 077 670**
**EP-A- 0 088 540**
**EP-A- 0 089 676**
**EP-A- 0 099 084**

**NATURE, vol. 295, 11 February 1982, Macmillan Journals Ltd; P.W. GRAY et al.: "Expression of human immune interferon cDNA in E. coli and monkey cells", pp. 503-508**

(73) Proprietor: **SUNTORY KABUSHIKI KAISHA**
**1-40, Dojimahama 2-chome**
**Kita-ku Osaka(JP)**

(72) Inventor: **Tanaka, Shoji**
**8-46, Minamikaneden-cho 1-chome**
**Suita Osaka 564(JP)**
Inventor: **Oshima, Takehiro**
**17-10-383, Bessho-hommachi**
**Takatsuki Osaka 569(JP)**
Inventor: **Nakasato, Hiroshi**
**13E-505, Misawa-cho**
**Ibaraki Osaka 567(JP)**
Inventor: **Noguchi, Teruhisa**
**8-11, Kugenuma-kaigan 2-chome**
**Fujisawa Kanagawa 251(JP)**

(74) Representative: **Burford, Anthony Frederick et al**
**W.H. Beck, Greener & Co. 7 Stone Buildings Lincoln's Inn**
**London WC2A 3SZ(GB)**

NUCLEIC ACIDS RESEARCH, vol. 8, no. 18, 1980, IRL Press Ltds., London, GB; D.V. GOEDDEL et al.:"Synthesis of human fibroblast interferon by E, coli", pp. 4057-4074

NUCLEIC ACIDS RESEARCH, Symposium Series, no. 11, 1982, IRL Press Ltd., Oxford, GB; S. TANAKA et al.:"Expression in Escherichia coli of chemically synthesized gene for a human immune interferon", pp. 29-32

NUCLEIC ACIDS RESEARCH; vol. 11, no. 6, March 1983, IRL Press Ltd., Oxford, GB; S. TANAKA et al.:"Expression in Escherichia coli of chemically synthesized gene for the human immune interferon", pp. 1707-1723

NUCLEIC ACIDS RESEARCH, 10(5), 1982, S. TANAKA et al., pp. 1741-1753

## Description

This invention relates to the production of a human gamma-interferon-like polypeptide (hereinafter abbreviated to γ-IFN).

The chemically synthesized DNA molecule as disclosed herein is characterized by the DNA sequence coding for a polypeptide substantially agreeing in the amino acid sequence and composition with human gamma-interferon and having the immunological or biological activity of human gamma-interferon.

Interferon includes three classes, namely alpha, beta and gamma, of proteins reportedly capable of inducing an antiviral condition in cells also showing antitumor activity.

It is known that γ-IFN can be produced by the genetic engineering technique using a gene prepared by reverse transcription of a messenger RNA in the presence of a reverse transcriptase (Nature, 295 , 503, 1982). However, this technique is disadvantageous, among others, in that, since the gene sequence is defined by the messenger RNA, it is impossible to select codons adequate to host cells.

Chemical synthesis of the γ-IFN gene may presumably overcome the above disadvantage and also may lead to construction of a chimeric polypeptide through addition of arbitrary restriction enzyme cleavage sites. However, the synthesis of said gene is not easy since it is composed of several hundreds of base pairs.

The present inventors endeavored to overcome this difficulty and succeeded in synthesizing a γ-IFN gene. Thus, they developed a method of producing γ-IFN by the genetic engineering technique using said gene.

The present invention thus provides a method of producing human gamma-interferon-like polypeptide which comprises cultivating in a nutrient medium host cells transformed with a plasmid with a polydeoxyribonucleotide, which has a translation-initiating codon at the 5'-end followed by a nucleotide sequence consisting of

```
1                                        10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

          20                                      30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                            40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

    50                                      60
CAG TCT CAG ATC GTT TCT TTC TAC TTC AAG CTG TTC AAA AAC TTC AAG
GTC AGA GTC TAG CAA AGA AAG ATG AAG TTC GAC AAG TTT TTG AAG TTC

                    70                                      80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                                90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT
```

```
                      100                                                   110
AAG CTT ACT AAC TAC TCT GTT ACT GAC CTT AAT GTA CAG CGT AAA GCT
TTC GAA TGA TTG ATG AGA CAA TGA CTG GAA TTA CAT GTC GCA TTT CGA


                                             120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT


        130                                          140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA


        146
TCT CAG
AGA GTC
```

```
                1                            1
wherein      TGC      may be replaced by   TGT
             ACG                           ACA
```

coding for the amino acid sequence

```
        1                                        10
    Cys Tyr Cys Gln Asp Pro Tyr Val Lys Glu Ala Glu
                                 20
  Asn Leu Lys Lys Tyr Phe Asn Ala Gly His Ser Asp Val
                     30
  Ala Asp Asn Gly Thr Leu Phe Leu Gly Ile Leu Lys Asn
      40                                      50
  Trp Lys Glu Glu Ser Asp Arg Lys Ile Met Gln Ser Gln
                                 60
  Ile Val Ser Phe Tyr Phe Lys Leu Phe Lys Asn Phe Lys
                         70
  Asp Asp Gln Ser Ile Gln Lys Ser Val Glu Thr Ile Lys
          80                                      90
  Glu Asp Met Asn Val Lys Phe Phe Asn Ser Asn Lys Lys
                                 100
  Lys Arg Asp Asp Phe Glu Lys Leu Thr Asn Tyr Ser Val
                         110
  Thr Asp Leu Asn Val Gln Arg Lys Ala Ile His Glu Leu
                 120
  Ile Gln Val Met Ala Glu Leu Ser Pro Ala Ala Lys Thr
  130                                      140
  Gly Lys Arg Lys Arg Ser Gln Met Leu Phe Arg Gly Arg

  Arg Ala Ser Gln
              140
```

and a subsequent nucleotide sequence coding for the termination of translation, inserted therein, and thereby causing formation of the polypeptide having the above amino acid sequence as a protein component in the culture broth. The invention also includes the above-mentioned polydeoxyribonucleotide, plasmids incorporating such a polydeoxyribonucleotide, and Escherichia coil strains transformed with such a plasmid.

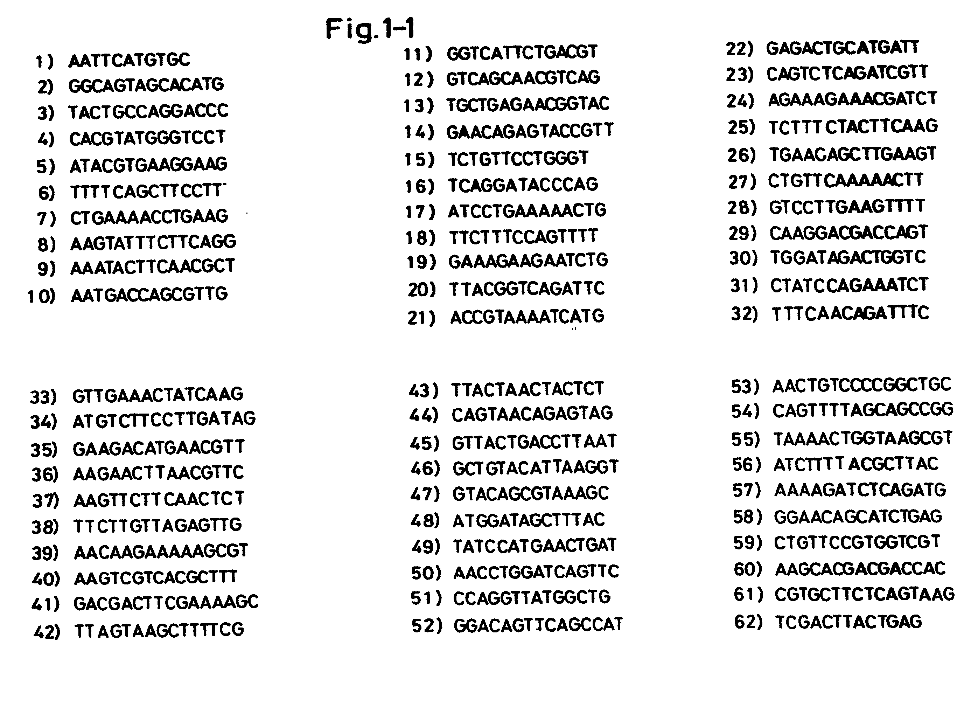

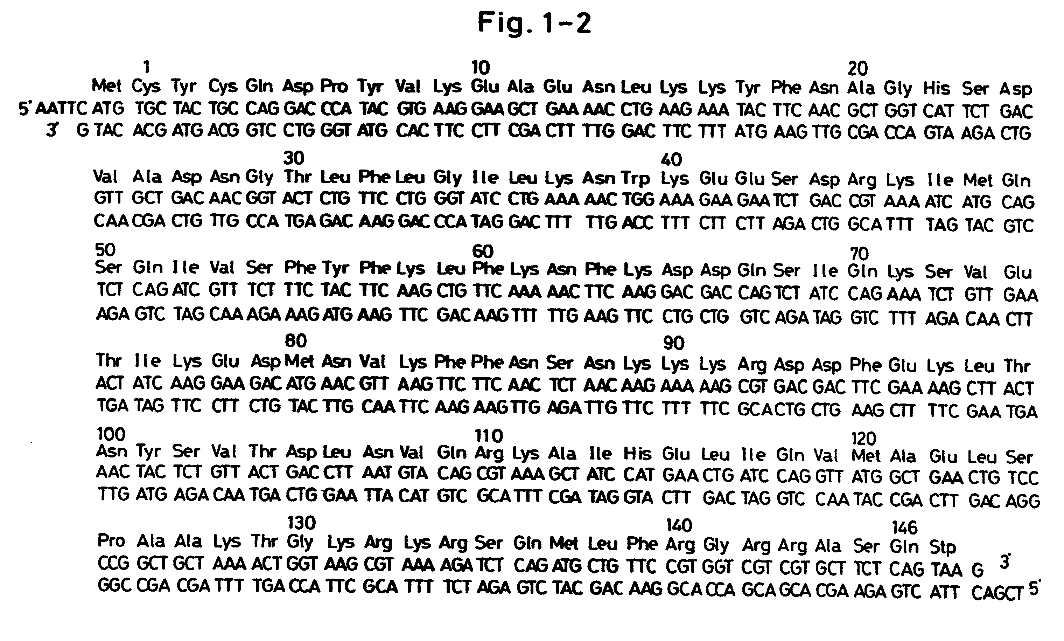

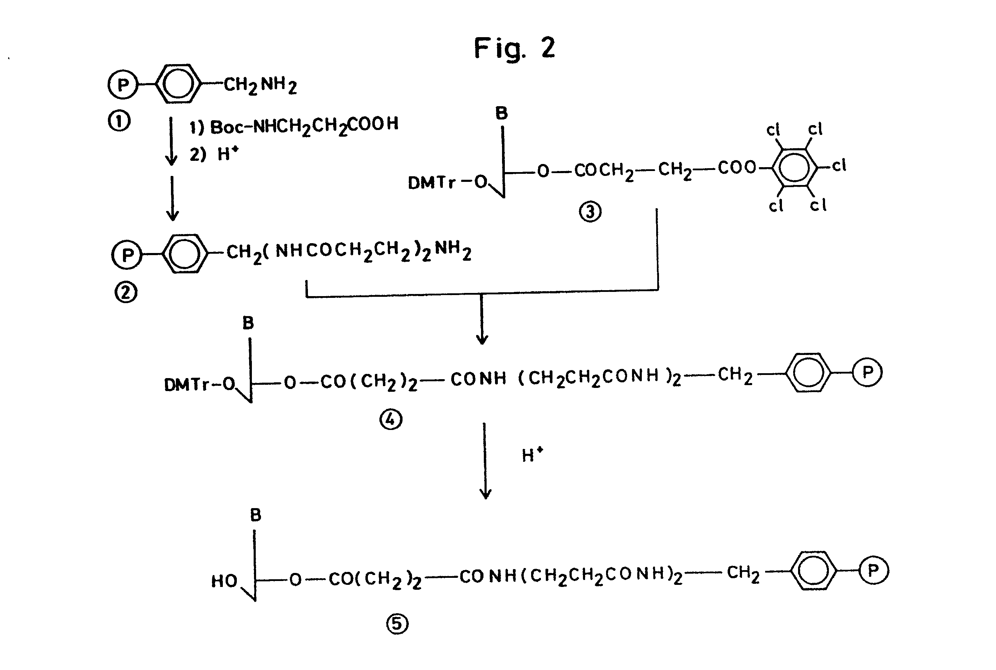

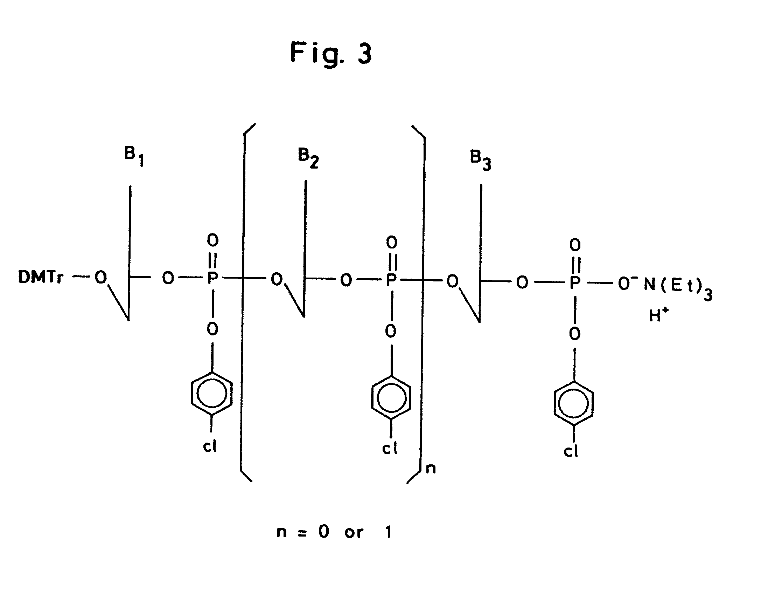

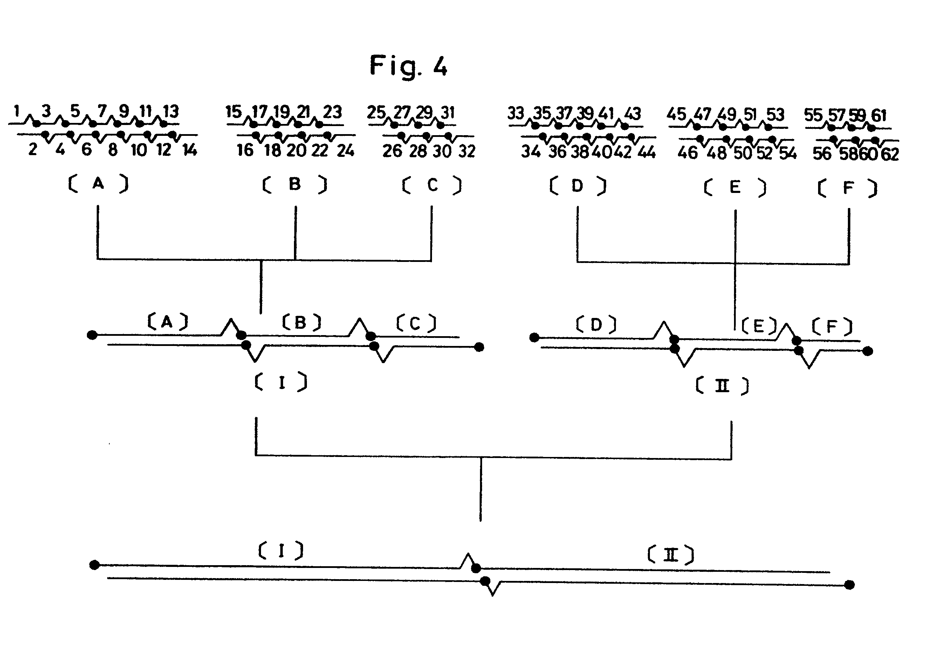

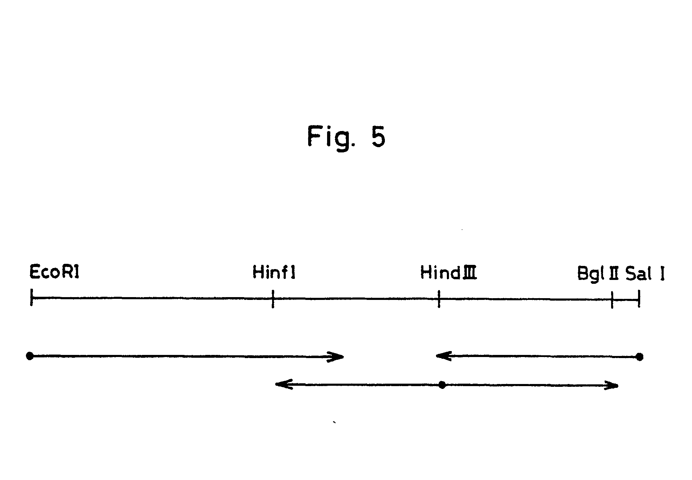

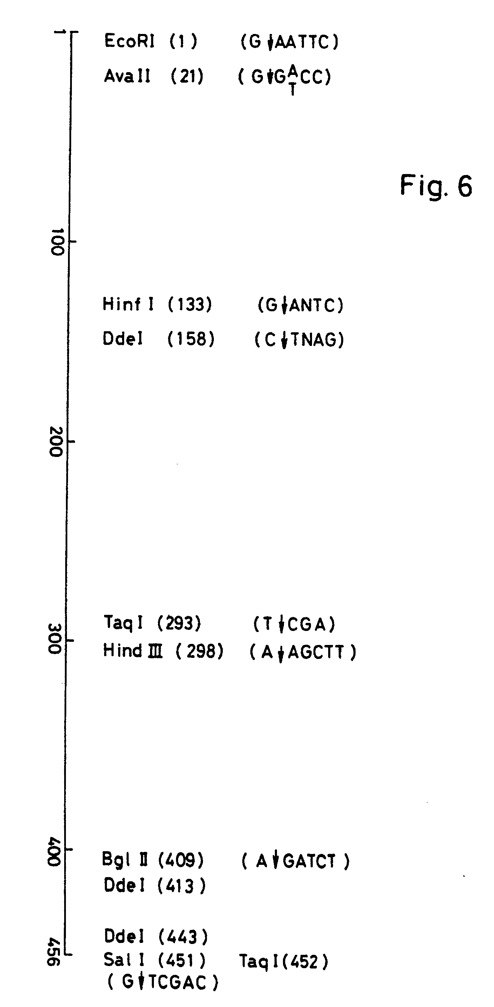

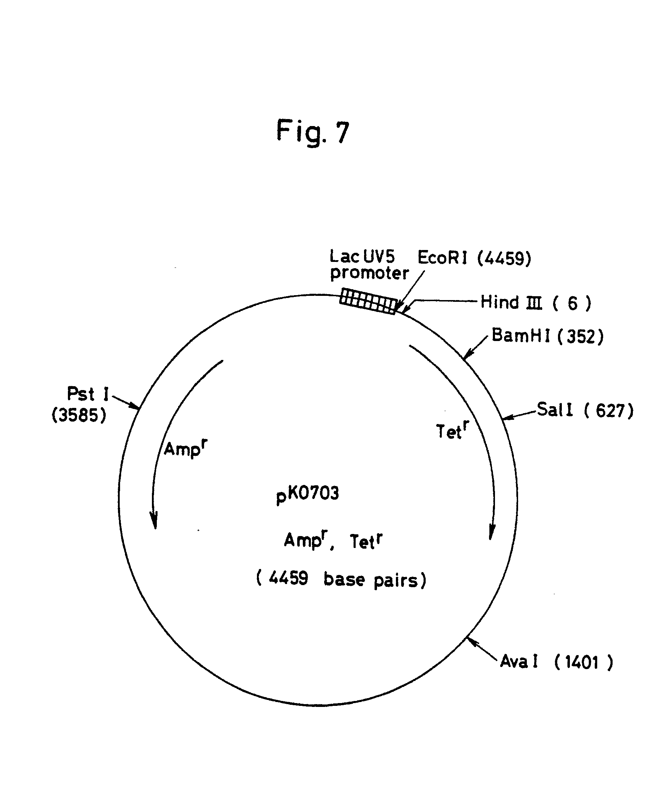

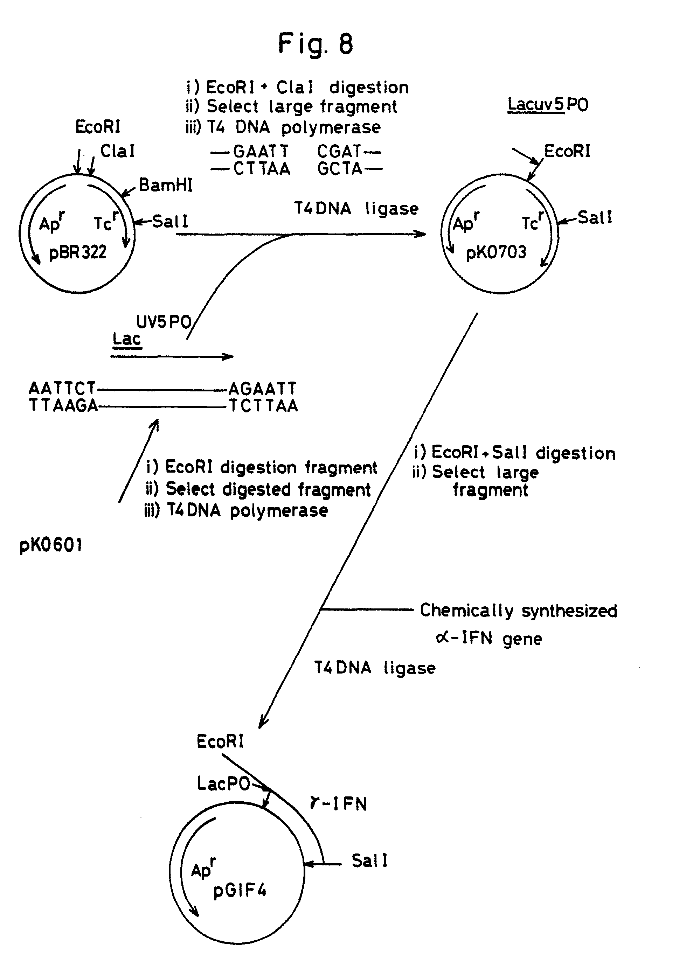

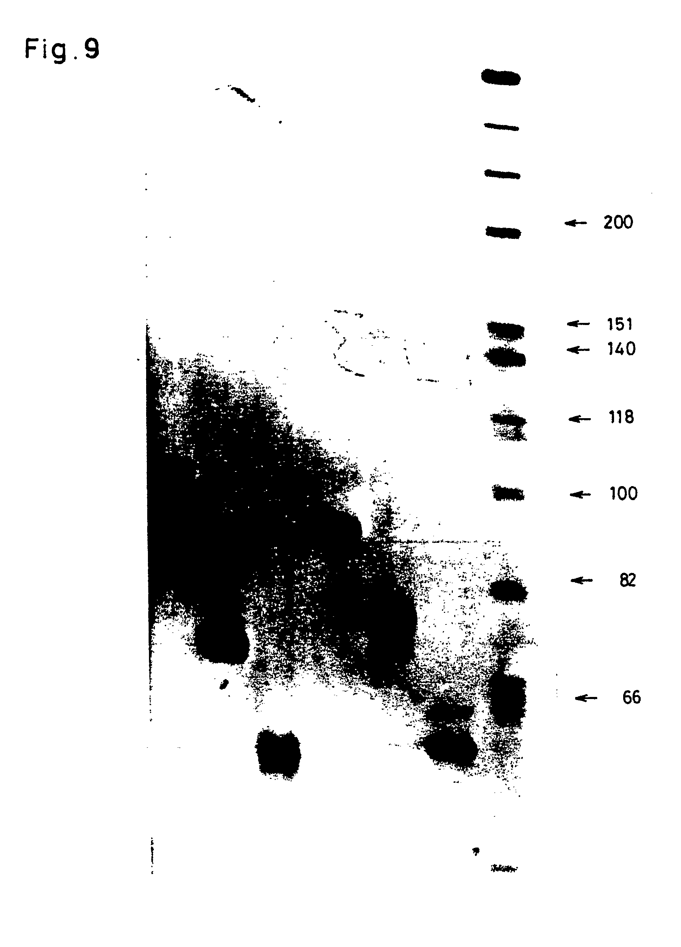

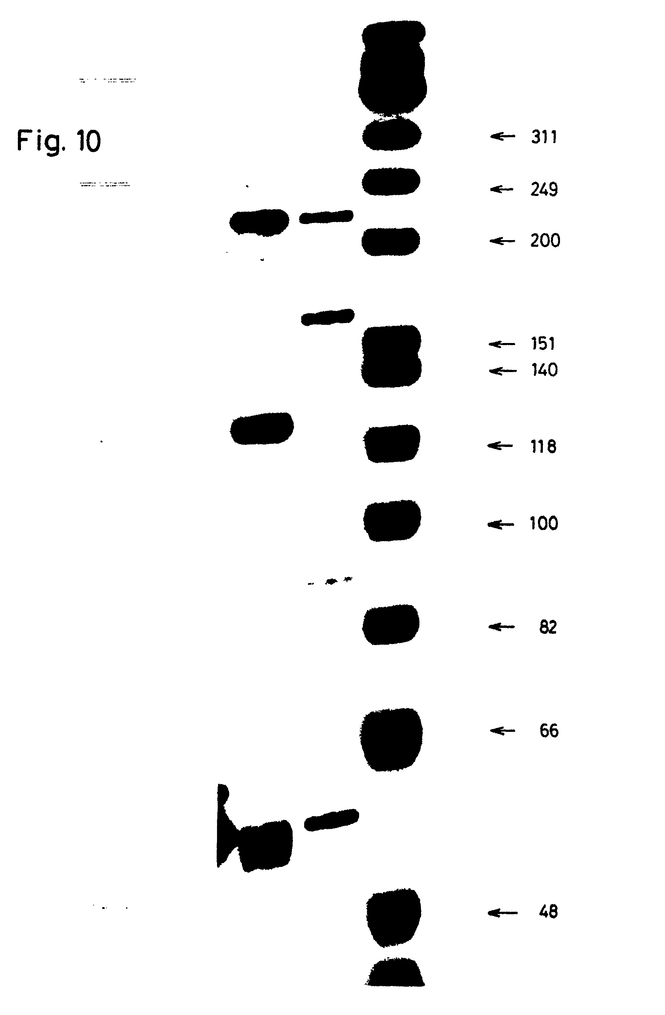

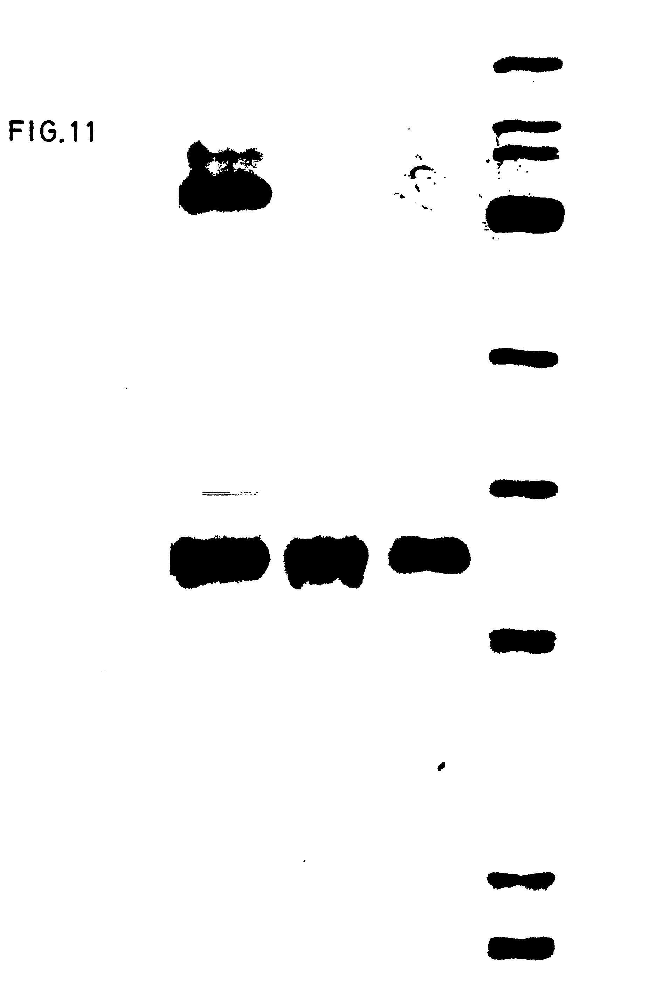

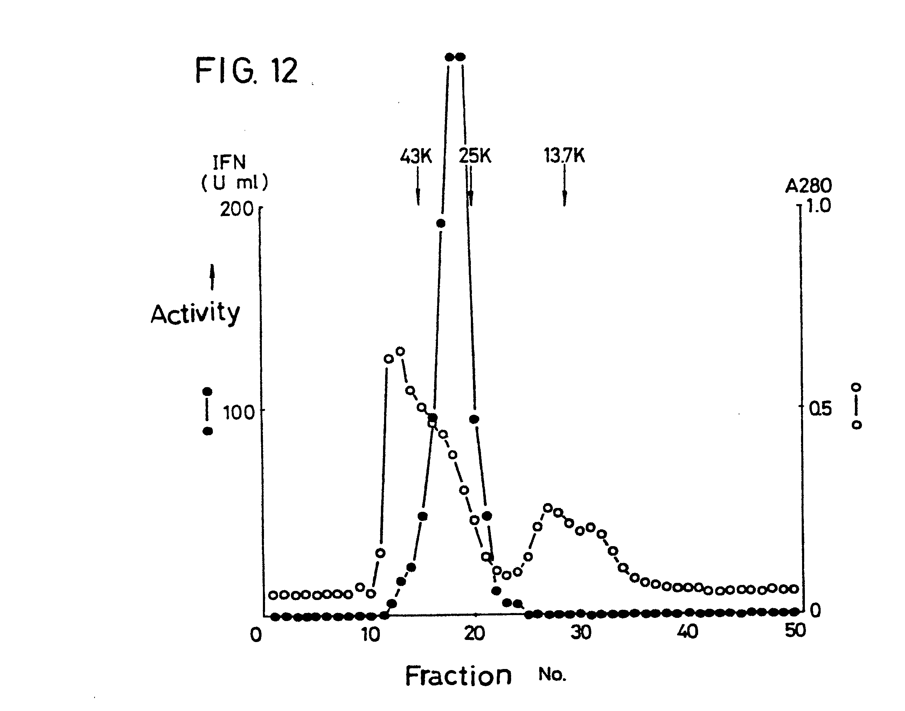

Referring to the drawings, Figs 1 to 5 and Figs. 8 to 11 are given by way of supplement to the

EP 0 095 350 B1

description of the example. Thus, sequence of each of 62 oligodeoxyribonucleotides used for the synthesis of a synthetic γ-IFN gene, Fig.1-(2) shows the amino acid sequence of human γ-IFN and the nucleotide sequence of the γ-IFN gene, Figs. 2 and 3 illustrate the oligodeoxyribonucleotide synthesis reaction process, Fig. 4 shows the oligodeoxyribonucleotide ligation scheme, Fig. 5 illustrates the scheme of determining the base sequence of the γ-IFN gene synthesized, and Fig. 8 shows the process of construction of plasmid pGIF. Fig. 9 is the 14% polyacrylamide gel electrophoresis mapping for the polydeoxyribonucleotide blocks A. B. C. D. E and F synthesized and for the molecular weight marker M ( Hinf I digest of φ x 174RF), Fig. 10 is the 8% polyacrylamide gel electrophoresis mapping for each of the block products [I] and [II] of ligation according to the scheme shown in Fig. 4, and for the molecular weight marker M ( Hpa II digest of φ x 174RF), and Fig. 11 is the 6% polyacrylamide gel electrophoresis mapping for each of the above blocks [I] and [II], the synthetic γ-IFN gene and the molecular weight marker M ( Hpa II digest of pBR322 DNA). Fig. 6 is the restriction enzyme cleavage map for the synthetic γ-IFN gene and Fig. 7 is the restriction enzyme cleavage map for the plasmid pKO703, the numerical values in the parentheses representing the distances in number of base pairs from the Eco RI recognition site. Fig. 12 is a graphic representation of the molecular weight of each fraction obtained by gel filtration of the supernatant which in turn was obtained by centrifugation of disrupted Escherichia coli cells and which exhibited interferon activity.

A preferred example of the above-mentioned polydeoxyribonucleotide is the one having a sequence coding for the amino acid sequence 1-146 as shown in Fig. 1-(2) and having a translation-initiating codon, such as ATG, at the 5'-end and a translation-terminating codon, such as TAA, at the 3'-end.

Because ATG codes methionine, γ-IFN of present invention naturally comprises a peptide added with formyl-methionine or methionine at the 5'-end of the amino acid sequence 1-142 mentioned above, as shown in Fig. 1-(2).

For the sake of convenience in gene manipulation, it is preferable to add a nucleotide sequence capable of being cleaved with a restriction enzyme to each of the translation-initiating and translation-terminating codon endings. In a preferred embodiment, an Eco RI-cleavable nucleotide sequence is added to the 5'-end and a Sal I-cleavable nucleotide sequence to the 3'-end.

The above polydeoxyribonucleotide can be synthesized, for example, in the following manner. First, the 62 gene fractions (oligodeoxyribonucleotides) shown in Fig. 1-(1) are synthesized by the per se known modified phosphotriester method and solid phase method. Then, a complete γ-IFN gene [cf. Fig. 1-(2)] is constructed by ligation of these fragments using T4 DNA ligase. This synthetic gene contains restriction enzyme cleavage sites, as shown in Fig. 6. This is for the purpose of facillitating search of active site structures or construction of chimeric genes.

In the next place, the above gene is inserted into a vector plasmid. The following is an example of the insertion procedure.

First, a plasmid, pKO703, is constructed by inser ting the promoter region (99 base pairs) of the Escherichia coli lactose operon between the Eco RI and Cla I cleavage sites of pBR322. Then, a plasmid, pGIF, is constructed by inserting the synthetic gene shown in Fig. 1-(2) and having an Eco RI cleavage site on the 5'-end side and a SalI cleavage site on the 3'-end side between the Eco RI and Sal I cleavage sites of the plasmid pKO703 (cf. Fig. 8).

As the host cells, there may be used bacteria, yeasts and other fungi as well as cultured human and other animal cells and cultured plant cells.

The bacteria are, for example, of the genus Escherichia, Bacillus or Pseudomonas. More specifical examples are Escherichia coli, Bacillus subtilis, Bacillus stearothermophilus, Pseudomonas putida and Pseudomonas aeruginosa.

The yeasts are, for example, those belonging to the genus Saccharomyces. Saccharomyces cerevisiae is a preferred example.

A strain belonging to the genus Escherichia is transformed with the plasmid pGIF obtained in the manner mentioned above. When Escherichia coli WA802 is used as said strain a transformant strain named WA802/pGIF is obtained. This strain has ampicillin resistance (Amp$^r$) and tetracycline sensitivity (Tet$^s$), owing to which it can be isolated from nontransformants.

Upon inductive culture of the above transformant strain in a medium, such as L-broth, using isopropyl-B-D-thiogalactopyranoside, a substance having interferon activity is produced in the cells.

As an example of a clone of the strain WA 802/pGIF, the strain Escherichia coil WA802/pGIF4 has been given a code name SBMG105 and deposited with the Fermentation Research Institute, Agency of Industrial Science and Technology, under Deposit No. FERM BP 282.

A mode of practice of the present invention is described hereinbelow by way of example. Escherichia coli is sometimes referred to as E . coli .

5

Example

Chemical synthesis of gamma-IFN gene fragment

The chemical synthesis of 62 oligodeoxyribonucleotides respectively designated $F^1$ through $F_{62}$ in Fig. 1-1, except for the improvements mentioned hereinafter, was carried out basically by the solid phase method reported by Ken-ichi Miyoshi et al [Nucl. Acids Res. 8 , 5507 (1980)].

The outline of chemical synthesis of these oligodeoxyribonucleotides by the improved technology is as follows.

a) First, as shown in Fig. 2, the 3'-end mononucleoside unit was bound to an aminomethylated polystyrene resin. Thus, the aminomethylated polystyrene resin (1) shown in Fig. 2 is a resin crosslinked with 1% divinylbenzene, and the resin (2) was prepared by adding 2 units of beta-alanine to (1). Then, three different compounds (3) (B represents thymine, N-benzoylcytosine or N-isobutyrylguanine) were reacted with the above resin (2) to provide 3 different polystyrene resins (4) each carrying the 3'-end nucleoside.

The following is an example of synthesis of the same resin (4) relevant to N-isobutyrylguanine for B in Fig. 2.

Twenty grams of aminomethylated polystyrene•HCl (divinylbenzene 1%, 100-200 mesh, $NH_2$: 0.63 m mol/g) was swollen well with 100 ml of $CH_2Cl_2$ and N-t-BOC-beta-alanine (1.34 g, 15.1 m moles) was added. The condensation reaction was conducted in the presence of DCC (3.11 g, 15.1 m moles) at room temperature for 3 hours. The resin was filtered through a glass filter and washed twice with 200 ml portions of $CH_2Cl_2$, 3 times with 150 ml portions of DMF and finally 3 times with 100 ml portions of pyridine. Then, the resin was treated with 100 ml of 25% acetic anhydride-pyridine for 1 hour to acetylate the unreacted amino groups. Thereafter, the resin was treated with 120 ml of 20% $CF_3COOH$-$CH_2Cl_2$ at room temperature for 30 minutes to eliminate the t-BOC groups. The resin was washed with 200 ml of 5% triethylamine-$CH_2Cl_2$ and further washed 4 times with 100 ml portions of $CS_2Cl_2$. The above procedure was repeated for a second time to give the resin (2)of Fig. 2, i.e. the aminomethylated polystyrene resin having two $\beta$-alanine units. In 60 ml of DMF was suspended 10 g of the above resin [Fig. 2, (2)], followed by the addition of 11 m moles of compound (3) [Fig. 2, (3)] (where B means N-isobutyrylguanine) and 1 ml of triethylamine. The mixture was shaken at room temperature for 12 hours. After the reaction, the resin was separated by filtration, washed with DMF and, then, with pyridine, and finally treated with 150 ml of 10% phenyl isocyanate-pyridine for 3 hours to protect the unreacted amino groups. The resin was subsequently washed with pyridine-methanol and dried under reduced pressure in the presence of $P_2O_5$, whereby the resin (4) of Fig. 2 (B means N-isobutyrylguanine) was obtained.

Using a portion of this resin (Fig. 2, (4), where B is N-isobutyrylguanine), the amount of 3'-end nucleoside secured to 1 gram of the resin was determined by the assay method of M.J. Gait et al [Nucl. Acids Res. 8 , 1081 (1980)]. The result was 0.12 m mole. The resin (4) wherein B is thymine or N-benzoylcytosine was also prepared in the same manner as above. The amount of 3'-end nucleoside secured to 1 g of resin (4) was 0.126 m mole for B-thymine and 0.125 m mol for B-N-benzoylcytosine.

b) Using these three kinds of resin (4) and the 3'-phosphoric diester dimer block (Fig. 3, n = 0) and 3'-phosphoric acid diester trimer block (Fig. 3, n = 1), 62 oligodeoxyribonucleotides $F_1$ through $F_{62}$ having the specified predetermined base sequences. Thus, the resin (4) was treated with 10% trichloroacetic acid (briefly, TCA)-$CHCl_3$ to remove the dimethoxytrytyl group (briefly, DMTr) and, then, 4 equivalents of 3'-phosphoric acid diester dimer (Fig. 3, n = 0) or 3 equivalents of 3'-phosphoric acid diester trimer (Fig. 3, n = 1) per equivalent of the 3'-nucleoside unit secured to resin (5) was sequentially condensed with the aid of 20 to 50 equivalents of mesitylenesulfonyltetrazolide (MsTe). The excess 3'-phosphoric acid diester block and condensing agent could be easily removed by filtering the reaction mixture through a glass filter.

Then, to protect the unreacted 5'-hydroxy group, the resin was either treated with 10% acetic anhydride-pyridine at room temperature for 1 hour or treated with a mixture of 10 ml of 10% acetic anhydride and 20 mg of 4-dimethylaminopyhridine (DMAP) at room temperature for 30 minutes, whereby the unreacted 5'-hydroxy groups were acetylated. The resin was then treated with 10% TCA to remove the 5'-end DMTr group and, then, the next condensation reaction was conducted. The above procedure was repeated until the required length was obtained.

An exemplary sequence of synthesis is given below with reference to the $F_{16}$ fragment.

200 mg of resin (4) [Fig. 2, (4), B = N-isobutyrylguanine] was treated 3 times with 20 ml of 10% TCA-$CHCl_3$ for 30 seconds each to give the resin (5) free from DMTr. To this resin (5) was added 96 $\mu$ moles

of the 3'-phosphoric acid dimer block CA (Fig. 3, n = 0, $B_1$ = N-benzoylcytosine, $B_3$ = N-benzoyladenine) and the azeotropic distillation with 3 ml of pyridine was repeated 3 times. Then, the resin was suspended in 2 ml of pyridine and the condensation reaction using 1 m mole of the condensing agent MsTe was carried out at room temperature for 2 hours. After the reaction, the excess 3'-phosphoric acid dimer and condensing agent could be easily removed by filtering the reaction mixture through a glass filter and washing the resin well with pyridine. Then, to protect the unreacted 5'-hydroxy groups, the resin was suspended in 20 ml of 10% acetic anhydride-pyridine and the suspension was shaken at room temperature for 1 hour. After this reaction, the resin was separated by filtration through a glass filter and washed well with pyridine and $CHCl_3$ in the order mentioned. Then, the resin was treated 3 times with 20 ml of 10% $TCA$-$CHCl_3$ for 30 seconds each, whereby the DMTr group was removed from the resin. The resin was washed well with $CHCl_3$ and pyridine in that order. The above procedure was repeated until the required length was obtained. Thus, to synthesize the oligodeoxyribonucleotide of F-16, the next 3'-phosphoric acid dimer blocks CC, TA, GA and TC were sequentially employed. By these sequential condensation reactions, a completely protected oligodeoxyribonucleotide-resin having the base sequence of F-16 was obtained. The other frangments $F_1$ to $F_{15}$ and $F_{17}$ to $F_{62}$ were also produced in the same manner except that different combinations of nucleotides were employed.

c) The severence of the completely protected oligodeoxyribonucleotide having such a definite base sequence from the resin and the complete deprotection thereof were carried out by treating the resin with concentrated aqueous ammonia-pyridine (2:1, v/v) at $55°$ C for 10 hours and, then, with 80% acetic acid at room temperature for 10 minutes (Process A). However, the fragments $F_2$, $F_3$, $F_5$, $F_7$ to $F_{10}$, $F_{22}$ to $F_{24}$, $F_{26}$, $F_{29}$, $F_{30}$, $F_{33}$ to $F_{42}$, $F_{53}$ to $F_{55}$, and $F_{57}$ to $F_{62}$ could not be obtained under the above deprotection conditions. Therefore, these fragments were completely deprotected by the method of Reese et al. [Nucl. Acids Res. 9 , 4611 (1981)] (Process B).

This deprotection procedure is described below with reference to $F_{16}$ and $F_{26}$ fragments. Process A:

In a sealed tube, 50 mg of the $F_{16}$-resin was shaken with 1 ml of pyridine and 2 ml of concentrated aqueous ammonia at $55°$ C for 10 hours, at the end of which time the resin was filtered off and the filtrate was concentrated under reduced pressure. To the residue was added toluene and the azeotropic distillation was repeated to remove the pyridine. The residue was treated with 2 ml of 80% acetic acid at room temperature for 10 minutes, whereby a completely deprotected oligodeoxyribonucleotide was obtained.

Process B:

50 mg of the $F_{26}$-resin was treated with a solution (0.3 M) of 1,1,4,4-tetramethylguanidium 2-pyridinealdoximate in 3.5 ml of dioxane-water (7:1, v/v) at $40°$ C for 48 hours and, then, in a sealed tube, it was further treated with 3 ml of concentrated aqueous ammonia-pyridine (2:1, v/v) at $55°$ C for 10 hours. Thereafter, the $F_{26}$-resin was treated with 2 ml of 80% acetic acid at room temperature for 20 minutes to give a completely deprotected oligodeoxyribonucleotide.

d) The isolation and purification of the final product was carried out by high performance liquid chromatography (briefly, HPLC. Thus, ALTEX Model 110 A Liquid Chromatograph, a linear gradient method using Solvent A (20% $CH_3CN$-l m $MKH_2PO_4$, pH 6.5) and Solvent 9 (20% $CH_3CN$-500 mM $KH_2PO_4$, pH 6.5), and a Partisil 10 SAX (Whatman) column (4.6 dia. x 250 mm) were employed. The gradient was established by adding 3% of Solvent B to Solvent A at the intervals of 1 minute. Elution was carried out at $58°$ C and a flow rate of 2 ml/min. The fractions containing the desired compound were pooled, desalted by dialysis and lyophilized. The homogeniety of the 62 completely deprotected oligodeoxyribonucleotides $F_1$ through $F_{62}$ thus obtained was confirmed by labeling their 5'-ends with [gamma-$^{32}$P]ATP using $T_4$ polynucleotide kinase and carrying out polyacrylamide gel (20%) electrophoresis.

The base sequence of each oligodeoxyribonucleotide was confirmed by the two-dimensional mapping method [Bambara, J.E. et al (1974) Nucl. Acids. Res. 1 , 331]. Thus, the 5'-end was labeled with[gamma-$^{32}$P]ATP using $T_4$ polynucleotide kinase and, then, partial hydrolysis was carried with nuclease. This partial hydrolysate was subjected to cellulose acetate electrophoresis (pH 3.5) and DEAE-cellulose homochromatography.

Ligation of gamma-IFN gene fragments

Using the 62 oligodeoxyribonucleotide fragments (1 to 62) synthesized in the above manner, the gene (Fig. 1-2) corresponding to γ-IFN consisting of 450 base pairs were ligated according to the ligation schema shown in Fig. 4. Details of the procedure are set forth below.

[A] to [F] blocks were synthesized. Excepting the 5'-end fragments of each block (1, 14, 15, 24, 25, 32, 33, 44, 45, 54, 55, 62), 1 n mole of each of the remaining 50 fragments was dissolved in 22 $\mu$l of distilled water and the solution was heated at 90°C for 2 minutes and, then, immediately cooled to 0°C. To this aqueous solution was added 5 $\mu$Ci of [$\gamma$-$^{32}$P] ATP (5100 Ci/m mole). It was then adjusted to give a 70 mM Tris-HCl (ph 7.5)-10 mM MgCl$_2$ kinase buffer solution. Then, 3 units of polynucleotide kinase was added to make a total of 30 $\mu$l. By conducting the reaction at 37°C for 15 minutes, the 5'-ends were labeled with $^{32}$P. Then, to phosphorylate all the 5'-ends, 4 n moles of ATP and 3 units of polynucleotide kinase were further added and the reaction was continued at 37°C for 45 minutes. The reaction was terminated by heating the mixture at 90°C for 5 minutes. 2.1 $\mu$g each of the above phosphorylated fragments 2 through 13 and unphosphorylated fragments 1 and 14 (A Block), phosphorylated fragments 16 through 23 and unphosphorylated fragments 15 and 24 (B Block), phosphorylated fragments 26 through 31 and unphosphorylated fragments 25 and 32 (C Block), phosphorylated fragments 34 through 43 and unphos phorylated fragments 33 and 44 (D Block), phosphorylated fragments 46 through 53 and unphosphorylated fragments 45 and 54 (E Block), and phosphorylated fragments 56 through 61 and unphosphorylated fragments 55 and 62 (F Block) were respectively mixed and dialyzed in a dialysis tube against distilled water at 4°C for 16 hours to remove the unreacted ATP and kinase buffer components. This DNA dialyzate for each of A to F blocks was concentrated to dryness and dissolved in 14.5 $\mu$l of ligase buffer (20 mM Tris-HCl, pH 7.6, 10 mM MgCl$_2$). This solution was put in a 40 $\mu$l fine-gauge glass tube and after both ends of the tube were sealed, it was heated in a 16.5 mm test tube containing 20 ml of water at 95°C for 2 minutes. Then, the test tube as a whole was allowed to stand at room temperature for 2 hours to aneal the DNA fragment. It was then placed at 0°C. This DNA solution was taken out of the fine-gauge glass tube and made into a ligase buffer containing 0.4 mM of ATP and 10 mM of DTT. Then, 30 units of T4 DNA ligase was added to make a total volume of 80 $\mu$l and the reaction was allowed to proceed at 15°C for 16 hours. A portion of the reaction mixture was taken and subjected to electrophoresis with 14% polyacrylamide gel containing 7 M of urea and the state of ligation was investigated by radioautography. As a result, the largest ligation reaction products were found to agree in size with the desired products for 6 blocks A through F. Then, the entire amount of the above ligation product was fractionated by electrophoresis through 14% polyacrylamide gel containing 7 M of urea. After the positions of A through F were confirmed by radioautography, the gel portions corresponding to the positions were cut out and placed in dialysis tubes. Each tube was filled with 9mM Tris-borate buffer (pH 8.3, 0.25 mM EDTA added) (elution buffer) and after closing both ends of the tube, electrophoresis was conducted in the elution buffer at a constant voltage of 200-V for 3 hours to elute the DNA in the gel. The DNA was taken out from the dialysis tube and lyophilized. The lyophilisate was dissolved in 100 $\mu$l of 0.3 M sodium acetate (pH 7.0), and two volumes of ethanol were added. The mixture was allowed to stand at -80°C for 30 minutes, at the end of which time it was centrifuged to recover the DNA as a sediment. The DNA sediments for A through F blocks were dissolved in 20 $\mu$l of distilled water and a 1 $\mu$l portion of the solution was investigated by electrophoresis with 14% polyacrylamide gels containing 7 M of urea. As shown in Fig. 9, substantially uniform bands corresponding to the sizes of DNAs for the respective blocks [A] through [F] were obtained. The DNA solution for each of A through F blocks was adjusted to the composition of the kinase buffer, and 2 n moles of ATP and 6 units of polynucleotide kinase were added. This reaction was conducted at 37°C for 1 hour, whereby the 5'-ends of the blocks were phosphorylated. The DNA for [A], [B] and [C] blocks (20 p moles each) were mixed and ATP and DTT were added in the concentrations of 0.4 mM ATP and 10 mM DTT and to make a total of 50 $\mu$l. Then, using 8 units of T4 DNA ligase, the mixture was reacted at 15°C for 16 hours. The DNA's for [D], [E] and [F] blocks were also mixed in the proportions of 5 p moles each and the ligation reaction was conducted at 15°C for 16 hours. After the above reaction, a portion of each reaction product was taken and the state of ligation was examined by electrophoresis with 8% polyacrylamide gels containing 7 M of urea. As shown in Fig. 10, it was found that [I] and [II] blocks corresponding to 225 bp had been synthesized. Then, the entire amount of the above reaction mixture was fractionated by electrophoresis through 8% polyacrylamide gels containing 7 M of urea. After the position of the 225 base-pair DNA was confirmed by radioautography, the corresponding portion of the gel was cut out and the DNA was eluted by the above-mentioned electro phoretic elution method. The DNA solution was lyophilized and dissolved in 100 $\mu$l of 0.3 M sodium acetate (pH 7.0), followed by addition of 2.5 volumes of ethanol, whereby the DNA was precipitated. To this DNA were added 0.5 n mole of ATP and 3 units of polynucleotide kinase and the reaction conducted at 37°C for 1 hour in the same manner as described hereinbefore. The DNA's (1 p mole each) of blocks [I] and [II] were mixed and adjusted to the ligase buffer composition. Then, ATP and DTT were added to the mixture in the concentrations of 0.4 mM ATP and 10 mM DTT to make a total of 20 $\mu$l. Using 5 units of T4 DNA ligase, the ligation reaction was conducted at 15°C for 16 hours. After the reaction, a portion of the reaction mixture was taken and the state of ligation was investigated by electrophoresis with 6% polyacrylamide gel containing 7 M of urea. The result is shown in Fig. 11. In Fig. 11, 1 and 11 stand for the isolated and purified products for [I] and [II] blocks, respectively.

Further, G denotes the result of ligation of the total $\gamma$-IFN gene, indicating that a DNA molecule corresponding to 450 base pairs was obtained.

To the whole amount of the above reaction mixture was added 1/10 volume of 3 M sodium acetate, followed by addition of 2.5 volumes of ethanol, whereby the DNA was recovered as a precipitate. This precipitate was dissolved in 100 $\mu$l of TA buffer (33 mM Tris-acetate, pH 7.9, 66 mM potassium phosphate, 10 mM magnesium acetate, 0.5 mM DTT), and 20 units of restriction enzyme Eco RI and 20 units of restriction enzyme Sal I were added. The reaction was conducted at 37°C for 1 hour. To the reaction mixture was added 10 $\mu$l of 0:3 M sodium acetate and, then, 2.5 volumes of ethanol was further added, whereby the DNA was recovered as a precipitate. This DNA was dissolved in a solution containing 80% formamide, 10 mM NaOH and 1 mM EDTA and the solution was subjected to electrophoresis with 6% polyacrylamide gels containing 7 M of urea. After confirming 450 base pairs by radioautography, the corresponding gel was cut out and put in a dialysis tube. The tube was filled with the elution buffer and after sealing, electrophoresis was conducted in the same buffer at a constant voltage of 200 V for 3 hours, whereby the DNA in the gel was eluted. The DNA solution was taken out from the dialysis tube and lyophilized. The lyophilisate was dissolved in 100 $\mu$l of 0.3 M sodium acetate (pH 7.0) and 2.5 volumes of ethanol was added, whereby the DNA was recovered as a precipitate. By the above procedure was obtained about 0.05 p mole of $\gamma$-IFN gene. This DNA was dissolved in 10 $\mu$l of the kinase buffer and 2 units of polynucleotide kinase and 0.1 n mole of ATP were added. The reaction was conducted at 37°C for 1 hour to provide a DNA for transformation.

Construction of pGIF plasmid

For the expresion of the chemically synthesized gene as a complete protein in Escherichia coli , an Escherichia coli plasmid pKO 703 into which the $\gamma$-IFN gene can be inserted downstream of the UV5 promoter operator region of the Escherichia coli lactose operon was constructed, and the $\gamma$-IFN gene was cloned in this pKO703 plasmid.

The details of construction of the series of plasmids employed in this experiment are set forth below (Fig. 8).

(1) Construction of pKO703 plasmid

The plasmid pKO703 containing the UV5 promoter-operator region of E . coli lactose operon was constructed by the following procedure.

(A) Preparation of pBR 322 plasmid with the EcoRI
and ClaI cleavage sites filled in

pBR 322 DNA (1.5 $\mu$g) was cleaved at 37°C for 60 minutes with 5 units each of the restriction enzymes EcoRI and Cla I in 20 $\mu$l of 1 x TA mixture (containing 33 mM Tris-acetate buffer, pH 7.6, 66 mM potassium acetate, 10 mM magnesium acetate and 0.5 mM dithiothreitol). The reaction mixture was heated at 65°C for 30 minutes so as to inactivate the enzymes. Addition of 2.5 volumes of cold ethanol gave a DNA precipitate. The DNA precipitate was suspended in 40 $\mu$l of T4 DNA polymerase solution and heated at 65°C for 10 minutes, followed by addition of 5 $\mu$l each of dTTP, dATP, dCTP and dGTP from the respective 4 mM solutions and 5 $\mu$l of 100 mM $\beta$-mercaptoethanol. Then, the mixture was reacted with 1 unit of T4 DNA polymerase at 37°C for 60 minutes. The reaction mixture was heated at 65°C for 30 minutes to inactivate the enzyme and precipitated with ethanol. Finally, the DNA precipitate was dissolved in 15 $\mu$l of distilled water.

(B) Preparation of the DNA in the Lac UV5 promoter-
operator region with the EcoRI cleavage sites
filled in

To separate the UV5 promoter-operator region of lactose operon, the following experiment was conducted.

Plasmid pKO601 is a plasmid having the Lactose operon promoter operator region between two Eco RI cleavage sites.

80 $\mu$g of this plasmid pKO601 was treated with 150 units of restriction enzyme Eco RI in 500 $\mu$l of 1 x TA mixture at 37°C for 60 minutes. The reaction mixture was subjected to 10% polyacrylamide gel electrophoresis. Thus, the DNA fragment of the promoter-operator region consisting of 95 base pairs and having Eco RI cohesive ends was separated by gel electrophoresis. (For details of the separation procedure, see Japanese Patent Application No. 163303/1981, Examples,the disclosure of which is republished in "Nucleic Acids Research 10(5), 1741, 1982.)

After ethanol precipitation, the DNA precipitate was dissolved in 40 $\mu$l of T4 DNA polymerase solution and the solution was heated at 65°C for 30 minutes and, then, quenched. Thereafter, 5 $\mu$l each of dATP, DTTP, dCTP and dGTP from the respective 4 mM solutions as well as 5 $\mu$l of 100 mM B-mercaptoethanol was added. The mixture was reacted using 10 units of T4 DNA polymerase at 37°C for 60 minutes. The reaction mixture was heated at 65°C for 30 minutes to inactivate the enzyme and the DNA was precipitated with ethanol. Finally, the DNA precipitate was dissolved in 20 $\mu$l of distilled water.

(C) Ligation of the plasmids prepared in (A) and (B)
to the lactose UV5 promoter-operator region, and
transformation thereof into Escherichia coli

Using 0.8 $\mu$g of the pBR 322 DNA with Eco RI and Cla I cleavage sites filled in as prepared in (A), 0.8$\mu$g of the lactose operon UV5 promoter-operator region DNA as prepared in (B), and 2.5 units of T4 DNA ligase, the ligation reaction was conducted at 16°C for 20 hours. (This ligation reaction was conducted in accordnace with the Examples of Japanese Patent Application No. 163303/1981).

After the ligation, the DNA was precipitated from ethanol and dissolved in 10 $\mu$l of distilled water. Using this DNA solution, transformation of Escherichia coli K12 WA802 was carried out. (This transformation was carried out in accordance with the method described in Japanese Patent Application No. 163303/1981.)

The transformant was cultivated on a M9S-X-gal medium [ NaCl , 5 g; NH$_4$Cl , 1 g; Na$_2$HPO$_4$, 5.94 g, KH$_2$PO$_4$, 3 g (pH 7.0), MgSO$_4$, 0.2 g; casamino acid, 2 g; glucose, 5 g; 5-bromo-4-chloro-3-indoyl-$\beta$-D-galactopyranoside, 400 mg; agar,15 g; water, 1000 ml; hereinafter briefly, M9S-X-gal medium] containing 40 $\mu$g/ml of ampicillin, and 10 ampicillin-resistant, deep blue colonies were picked up and designated as WA802/pKO701~WA802/pKO710.

The recognition site of restriction enzyme Cla I on plasmid pBR 322 lies within the promoter region of the tetracycline-resistant gene and if another DNA fragment is inserted between Eco RI and Cla I recognition sites on pBR 322 plasmid, Escherichia coli having such plasmid becomes tetracycline-sensitive. However, it is expected that if the DNA fragment in the UV5 promoter-operator region of the lactose operon is inserted with the desired directionality, the transformant having the plasmid will become tetracycline-insensitive.

Therefore, the WA802/pKO701 through WA802/pKO710 strains obtained above were cultivated by single colonization on the ordinary nutrient agar medium containing 10 $\mu$g/ml of tetracycline to examine their tetracycline sensitivity. It was found that WA 802/pKO701, WA802/pKO703, WA802/pKO706 and WA802/pKO709 were tetracycline-insensitive. Therefore, plasmid DNA was separated from each of these strains and 2 $\mu$g of DNA was reacted in 1 x TA mixture with 5 units of restriction enzymes Eco RI and Hpa II at 37°C for 60 minutes and the directionality of the lactose operon promoter-operator region was investigated by 10% polyacrylamide gel electrophoresis. It was found that in the case of WA 802/pKO703 and WA802/pKO709, the promoter-operation region had been inserted in the desired orientation. This pKO703 plasmid was used in the cloning of the $\gamma$-IFN gene. The plasmid pKO703 has Eco RI recognition site downstream in the transcription direction from the promoter, with the tetracycline-resistant gene of pBR 322 remaining intact on the plasmid, the gene having the cohesive ends of a restriction enzyme for cleaving the tetracycline-resistant structural gene, such as Eco RI, Sal I and BamHI obtained chemically or from a plasmid, can be easily introduced by ligation into the plasmid pKO703 and the desired clone can be easily selected as an ampicillin-resistant, tetracycline-sensitive strain.

(2) Construction of plasmid pGIF$_4$

In 30 $\mu$l of 100 mM tris-HCl buffer (pH 7.3)-5 mM MgCl$_2$-50 mM Nacl, 15 $\mu$g of plasmid pKO703 was reacted with 10 units each of restriction enzymes Eco RI and Sal I at 37°C for 60 minutes. The reaction mixture was heated at 65°C for 30 minutes to inactivate the enzymes and, then, subjected to 0.7% agarose gel electrophoresis. By electrophoresis, 3.8 kb of DNA fragment was separated from the gel. After ethanol precipitation, the DNA was dissolved in 4.5 $\mu$l of distilled water. The resulting Eco RI/ Sal I cleaved fragment DNA (0.3 $\mu$g) and the 5'-OH end phosphorylated $\gamma$-IFN gene (24 $\mu$l) previously obtained were dissolved in T4 DNA ligase mixture and the solution was heated at 65°C for 10 minutes. After cooling with ice, 3 $\mu$l of 100 mM DTT and 3 $\mu$l of 4 mM ATP were added, and using 3 units of T4 DNA ligase, ligation was carried out at 16°C for 20 hours. After this ligation reaction, 2.5 volumes of cold ethanol was added, whereby the DNA was precipitated. To this precipitated DNA was added 10 $\mu$l of distilled water and the transformation of E. coli WA802 was conducted. The transformant was cultivated on an M9S-X-gal medium containing 40 $\mu$g/ml of ampicillin and ampicillin-resistant and deep blue colonies were picked and transferred to an ordinary nutrient agar medium containing 10 $\mu$g/ml of tetracycline by single colonization. Tetracycline-sensitive colonies were selected and designated as WA802/pGIF$_1$ through WA802/pGIF$_5$.

After separation of plasmid DNA's, each plasmid DNA was cleaved with restriction enzymes Eco RI and Sal I, Eco RI and Hind III, or Eco RI and Bgl II and the sizes of DNA fragments were measured by agarose gel electrophoresis and polyacrylamide gel electrophoresis, In this manner, WA802/pGIF₄ and WA802/pGIF₅ wherein the desired γ-IFN gene had been cloned were confirmed. In these plasmids pGIF₄ and pGIF₅, the start codon ATG of the γ-IFN gene is situated 10 base pairs downstream of the Shine-Dalgano DNA base sequence in the UV₅ promoter region of the lactose operon.

### Shine-Dalgano base sequence

```
5' - - AGGAAACAGAATTCATG
             |        |
       - - TCCTTTGTCTTAAGTAC
```

**EcoRI recognition site**

Identification of the base sequence of a γ-IFN gene

The base sequence in the region corresponding to the 450 base pair γ-IFN gene obtainable by the Eco RI/ Sal I cleavage of the pGIF₄ plasmid DNA was identified according to the schema shown in Fig. 5. The determination of the base sequence was carried out in accordance with the Maxam-Gilbert method (Proc. Natl. Acad. Sci., U.S.A., 74 , 560-564, 1977). It was confirmed that throughout all the 450 base pairs the sequence was as initially designed (Fig. 1-2).

(3) Expression and identification of a human gamma-interferon-lake polypeptide in the E . coli cell

In E. coli WA802/pGIF₄ and WA802/pGIF₅, the Y-IFN gene was inserted downstream of the promoter region of the lactose operon. Therefore, it was expected that the γ-IFN would be expressed under the control of the lactose operon. Accordingly, the expression and fixation of γ-IFN in the cells of E . coli WA802/pGIF₄ and WA802/pGIF₅ were carried out. Each of E . coli WA802/pGIF₄ and WA802/pGIF₅ strains was used to inoculate 2.0 ml of M9S-glycerol medium (NaCl, 5 g; NH₄Cl, 1 g; Na₂HPO₄, 5.94 g; KH₂PO₄, 3 g; MgSO₄•7H₂O , 0.2 g; glycerol, 2.5 g, casamino acid, 2 g; water, 1000 ml) containing 40 $\mu$g/ml of ampicillin and the inoculated medium was incubated at 37°C overnight. The resulting culture was transferred to 4 tubes of M9S-glycerol medium (5 ml) containing 40 $\mu$g/ml of ampicillin in a size giving an absorbance (OD 660 nm) of 0.1. Incubation was carried out at 37°C and when the OD 660 nm reached 0.8, isopopyl-$\beta$-D-thiogalactopyranoside (briefly, IPTG) as a lactose operon inducer was added to two of the 4 tubes at the final concentration of 1 mM, further followed by incubation at 37°C for 2.5 hours. The remaining two tubes were incubated without IPTG at 37°C for 2.5 hours. A 5 ml portion each of these cultures was centrifuged at 3,000 r.p.m. for 10 minutes, whereby cells were recovered as a sediment. Then, 0.5 ml of 1 x PBS (10 mM phosphate buffer pH 7.0, 150 mM NaCl) containing 1 mg/ml of bovine serum albumin was added to each of the two tubes, one containing IPTG and the other not containing IPTG and the cells were distrupted by ultrasonication. To each of the remaining 2 tubes, one containing IPTG and the other not containing IPTG, was added 0.5 ml of 1 x PBS containing 1 mg/ml of egg white lysozyme. The lysozyme treatment was conducted at 0°C for 40 minutes, followed by ultra-sonication. 0.5 ml of the disrupted cells were centrifuged at 25,000 r.p.m. for 30 minutes at 4°C and the supernatant was used for the activity assay of γ-IFN.

Determination of interferon activity

Using a Coaster's 96-well plastic multiplate and a Eagle's minimal medium containing 5% of fetal calf serum, a dilution series of interferon samples were prepared by multi-pipetting. Then, 100 $\mu$l of FL cells (5 x 10⁵ cells/ml) were added to each well and the cultivation was conducted at 37°C overnight in a 5% $CO_2$ gas atmosphere. The culture supernatant was discarded and 100 $\mu$l of Sindbis virus in a concentration 100 times as high as $TCID_{50}$ was added to each well and the incubation was carried out at 37°C overnight in a 5% $CO_2$ gas atmosphere. After 17 hours, the supernatant was discarded and the residue was stained with 1% crystal violet solution containing 20% of ethanol. As to interferon potency, the dilution factor of the interferon sample showing the intensity of stain a half that of the difference in the intensity of stain between the virus-free well and the interferon-free, virus-added well was expressed in laboratory units/ml. This assay system affords a sensitivity 7 to 25 times as high as $\alpha$-interferon for which an international standard is available.

Whether the interferon activity was certainly human gamma-interferon activity was confirmed immunologically using an anti-human gamma-interferon antibody. In this case, 10 $\mu$l of anti-human gamma-interferon antibody (with a sufficient activity to inactivate 2,000 units/ml of human gamma-interferon activity) was added to 50 $\mu$l of an interferon active sample, and after standing at room temperature for 3 hours, the mixture was centrifuged at 10,000 r.p.m. for 5 minutes. The supernatant was separated for use as a sample.

As shown in Table 1, the transformed E . coli WA802/pGIF₄ and WA802/pGIF₅ were confirmed to have produced a human gamma-interferon-like substance under the control of the lactose operon, while the non-transformant (WA802/pKO703) did not show this activity.

The E . coli WA802/pGIF₄ disrupted/centrifuged super-natant which showed interferon activity in the above example was added to Ultrogel AcA 54 (0.7 cm dia. x 47 cm high) and its molecular weight was estimated by gel permeation chromatography, Physiologically balanced salt solution (1 x PBS) was used to obtain 0.7 ml fractions. As standard reference proteins, ribonuclease A (mol. wt. 13,700), chymotrypsinogen A (mol. wt. 25,000) and ovalbumin (mol. wt. 43,000) were employed. Interferon activity was eluted between chymotrypsinogen A and cvalbumin and the molecular weight of the peak interferon activity under the above conditions was estimated to be about 30,000 (See Fig. 12).

## Table 1

### Results of Assay of Interferon Activity

A:  Samples prepared by adding BSA to the centrifugal supernatants of ultrasonicated cells

| | IPTG induction | Interferon activity laboratory units/ml | | Activity after treatment with anti-$\gamma$-interferon antibody | |
|---|---|---|---|---|---|
| | | Trial 1 | Trial 2 | Trial 1 | Trial 2 |
| WA802/pKO703 | – | 0 | 0 | N.T. | N.T. |
| (Control) | + | 0 | 0 | N.T. | N.T. |
| WA802/PGIF$_4$ | – | 0 | 0 | 0 | 0 |
| | + | 24 | 18 | 0 | 0 |
| WA802/pGIF$_5$ | – | N.T. | N.T. | N.T. | N.T. |
| | + | 768 | 842 | 0 | 0 |

N.T.:  Not tested

EP 0 095 350 B1

CLAIMS for the contracting states BE, CH, DE, FR, GB, IT, LI, LU, NL, SE

Claims

### B: The centrifugal supernatants of lysozyme-treated ultrasonicated cells

| | IPTG induction | Interferon activity labolatory units/ml | | Activity after treatment with anti-γ-interferon antibody | |
|---|---|---|---|---|---|
| | | Trial 1 | Trial 2 | Trial 1 | Trial 2 |
| WA802/pKO703 | − | 0 | 0 | N.T. | N.T. |
| (Control) | + | 0 | 0 | N.T. | N.T. |
| WA802/PGIF$_4$ | − | 0 | 0 | 0 | 0 |
| | + | 272 | 280 | 0 | 0 |
| WA802/pGIF$_5$ | − | N.T. | N.T. | N.T. | N.T. |
| | + | 192 | 280 | 0 | 0 |

N.T.: Not tested

14

EP 0 095 350 B1

1. A method of producing human gamma-interferon-like polypeptide which comprises cultivating in a nutrient medium host cells transformed with a plasmid with a polydeoxyribonucleotide, which has a translation-initiating codon at the 5'-end followed by a nucleotide sequence consisting of

```
1                                          10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

         20                                        30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                                           40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

    50                                        60
CAG TCT CAG ATC GTT TCT TTC TAC TTC AAG CTG TTC AAA AAC TTC AAG
GTC AGA GTC TAG CAA AGA AAG ATG AAG TTC GAC AAG TTT TTG AAG TTC

                           70                                        80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                                   90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT

         100                                       110
AAG CTT ACT AAC TAC TCT GTT ACT GAC CTT AAT GTA CAG CGT AAA GCT
TTC GAA TGA TTG ATG AGA CAA TGA CTG GAA TTA CAT GTC GCA TTT CGA

                           120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT

    130                                       140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA

    146
TCT CAG
AGA GTC
```

<div align="center">

wherein $\dfrac{1}{\substack{\text{TGC} \\ \text{ACG}}}$ may be replaced by $\dfrac{1}{\substack{\text{TGT} \\ \text{ACA}}}$

</div>

coding for the amino acid sequence

```
          1                                                  10
          Cys Tyr Cys Gln Asp Pro Tyr Val Lys Glu Ala Glu
                                      20
      Asn Leu Lys Lys Tyr Phe Asn Ala Gly His Ser Asp Val
                  30
      Ala Asp Asn Gly Thr Leu Phe Leu Gly Ile Leu Lys Asn
          40                                          50
      Trp Lys Glu Glu Ser Asp Arg Lys Ile Met Gln Ser Gln
                                          60
      Ile Val Ser Phe Tyr Phe Lys Leu Phe Lys Asn Phe Lys
                          70
      Asp Asp Gln Ser Ile Gln Lys Ser Val Glu Thr Ile Lys
              80                                          90
      Glu Asp Met Asn Val Lys Phe Phe Asn Ser Asn Lys Lys
                                          100
      Lys Arg Asp Asp Phe Glu Lys Leu Thr Asn Tyr Ser Val
                          110
      Thr Asp Leu Asn Val Gln Arg Lys Ala Ile His Glu Leu
                  120
      Ile Gln Val Met Ala Glu Leu Ser Pro Ala Ala Lys Thr
      130                                         140
      Gly Lys Arg Lys Arg Ser Gln Met Leu Phe Arg Gly Arg

      Arg Ala Ser Gln
```

and a subsequent nucleotide sequence coding for the termination of translation, inserted therein, and thereby causing formation of the polypeptide having the above amino acid sequence as a protein component in the culture broth.

2. A method as claimed in claim 1 wherein the host cells are of a strain belonging to the genus Escherichia.

3. A method as claimed in claim 2, wherein the host cells are of a strain belonging to the species Escherichia coli .

4. A method as claimed in claim 3, wherein the host cells transformed are of Escherichia coli K12.WA802.

5. A method as claimed in any preceding claim, wherein the gamma-interferon-like polypeptide is recovered from the culture broth.

6. A polydeoxypoynucleotide of the sequence,

```
1                                        10
TGC TAC TGC CAG GAC CCA TAC GTG AAG GAA GCT GAA
ACG ATG ACG GTC CTG GGT ATG CAC TTC CTT CGA CTT


                             20
AAC CTG AAG AAA TAC TTC AAC GCT GGT CAT TCT GAC GTT
TTG GAC TTC TTT ATG AAG TTG CGA CCA GTA AGA CTG CAA


                 30
GCT GAC AAC GGT ACT CTG TTC CTG GGT ATC CTG AAA AAC
CGA CTG TTG CCA TGA GAC AAG GAC CCA TAG GAC TTT TTG


         40                              50
TGG AAA GAA GAA TCT GAC CGT AAA ATC ATG CAG TCT CAG
ACC TTT CTT CTT AGA CTG GCA TTT TAG TAC GTC AGA GTC


                             60
ATC GTT TCT TTC TAC TTC AAG CTG TTC AAA AAC TTC AAG
TAG CAA AGA AAG ATG AAG TTC GAC AAG TTT TTG AAG TTC


                 70
GAC GAC CAG TCT ATC CAG AAA TCT GTT GAA ACT ATC AAG
CTG CTG GTC AGA TAG GTC TTT AGA CAA CTT TGA TAG TTC


         80                              90
GAA GAC ATG AAC GTT AAG TTC TTC AAC TCT AAC AAG AAA
CTT CTG TAC TTG CAA TTC AAG AAG TTG AGA TTG TTC TTT


                                 100
AAG CGT GAC GAC TTC GAA AAG CTT ACT AAC TAC TCT GTT
TTC GCA CTG CTG AAG CTT TTC GAA TGA TTG ATG AGA CAA


                     110
ACT GAC CTT AAT GTA CAG CGT AAA GCT ATC CAT GAA CTG
TGA CTG GAA TTA CAT GTC GCA TTT CGA TAG GTA CTT GAC


             120
ATC CAG GTT ATG GCT GAA CTG TCC CCG GCT GCT AAA ACT
TAG GTC CAA TAC CGA CTT GAC AGG GGC CGA CGA TTT TGA


130                                  140
GGT AAG CGT AAA AGA TCT CAG ATG CTG TTC CGT GGT CGT
CCA TTC GCA TTT TCT AGA GTC TAC GAC AAG GCA CCA GCA


         146
CGT GCT TCT CAG
GCA CGA AGA GTC
```

wherein $\dfrac{TGC}{ACG}^{1}$ may be replaced by $\dfrac{TGT}{ACA}^{1}$

7. A polydeoxyribonucleotide as claimed in claim 6, having in addition to said sequence a translation-initiating codon at the 5'-end and a translation-terminating codon at the 3'-end.

8. A polydeoxyribonucleotide as claimed in claim 7, having restricted enzyme-cleavable nucleotide sequences added to both ends.

9. A polydeoxyribonucleotide as claimed in claim 8 wherein the 5'-end side is cleavable with Eco RI and the 3'-end side with Sal I.

10. A plasmid with a polydeoxyribonucleotide as defined in any one of claims 6 to 9 inserted therein.

11. A strain belonging to Escherichia coli and transformed with a plasmid as claimed in claim 10.

CLAIMS for the contracting state HT

1. A method of producing human gamma-interferon-like polypeptide which comprises cultivating in a nutrient medium host cells transformed with a plasmid with a polydeoxyribonucleotide, which has a translation-initiating codon at the 5'-end followed by a nucleotide sequence consisting of

```
1                                                       10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

                        20                                      30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                                        40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

        50                                      60
CAG TCT CAG ATC GTT TCT TTC TAC TTC AAG CTG TTC AAA AAC TTC AAG
GTC AGA GTC TAG CAA AGA AAG ATG AAG TTC GAC AAG TTT TTG AAG TTC

                        70                                      80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                                        90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT

            100                                     110
AAG CTT ACT AAC TAC TCT GTT ACT GAC CTT ATT GTA CAG CGT AAA GCT
TTC GAA TGA TTG ATG AGA CAA TGA CTG GAA TTA CAT GTC GCA TTT CGA

                                120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT

        130                                     140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA

        146
TCT CAG
AGA GTC
```

```
        1                              1
wherein TGC may be replaced by TGT
        ACG                          ACA
```

coding for the amino acid sequence

```
    1                                       10
    Cys Tyr Cys Gln Asp Pro Tyr Val Lys Glu Ala Glu
                            20
    Asn Leu Lys Lys Tyr Phe Asn Ala Gly His Ser Asp Val
                    30
    Ala Asp Asn Gly Thr Leu Phe Leu Gly Ile Leu Lys Asn
        40                                      50
    Trp Lys Glu Glu Ser Asp Arg Lys Ile Met Gln Ser Gln
                                    60
    Ile Val Ser Phe Tyr Phe Lys Leu Phe Lys Asn Phe Lys
                        70
    Asp Asp Gln Ser Ile Gln Lys Ser Val Glu Thr Ile Lys
            80                                      90
    Glu Asp Met Asn Val Lys Phe Phe Asn Ser Asn Lys Lys
                                    100
    Lys Arg Asp Asp Phe Glu Lys Leu Thr Asn Tyr Ser Val
                            110
    Thr Asp Leu Asn Val Gln Arg Lys Ala Ile His Glu Leu
                    120
    Ile Gln Val Met Ala Glu Leu Ser Pro Ala Ala Lys Thr
    130                                     140
    Gly Lys Arg Lys Arg Ser Gln Met Leu Phe Arg Gly Arg

    Arg Ala Ser Gln
```

and a subsequent nucleotide sequence coding for the termination of translation, inserted therein, and thereby causing formation of the polypeptide having the above amino acid sequence as a protein component in the culture broth.

2.  A method as claimed in Claim 1 wherein the host cells are of a strain belonging to the genus Escherichia.

3.  A method as claimed in Claim 2, wherein the host cells are of a strain belonging to the species Escherichia coli .

4.  A method as claimed in Claim 3, wherein the host cells transformed are of Escherichia coli K12.WA802.

5.  A method as claimed in any preceding claim, wherein the gamma-interferon-like polypeptide is recovered from the culture broth.

6.  A method as claimed in any preceeding claim wherein the said nucleotide sequence has restricted enzyme cleavable nucleotide sequences added to both ends.

7.  A method as claimed in Claim 6, wherein the 5'-end side is cleavable with Eco RI and the 3'-end side with Sal I.

**Revendications**

Revendications pour les Etats contractants BE, CH, DE, FR, GB, IT, LI, LU, NL, SE

1.  Procéde de production d'un polypeptide similaire à l'interféron γ humain, qui consiste à cultiver dans un milieu nutritif des cellules hôtes transformees par un plasmide ayant un polydésoxyribonucléotide qui a un codon d'initiation de la traduction à l'extrémité 5' suivi d'une séquence de nucléotides consistant en

```
     1                                           10
     TGC TAC TGC CAG GAC CCA TAC GTG AAG GAA GCT GAA AAC CTG AAG AAA
     ACG ATG ACG GTC CTG GGT ATG CAC TTC CTT CGA CTT TTG GAC TTC TTT

                    20                                           30
     TAC TTC AAC GCT GGT CAT TCT GAC GTT GCT GAC AAC GGT ACT CTG TTC
     ATG AAG TTG CGA CCA GTA AGA CTG CAA CGA CTG TTG CCA TGA GAC AAG

                                        40
     CTG GGT ATC CTG AAA AAC TGG AAA GAA GAA TCT GAC CGT AAA ATC ATG
     GAC CCA TAG GAC TTT TTG ACC TTT CTT CTT AGA CTG GCA TTT TAG TAC

            50                                       60
     CAG TCT CAG ATC GTT TCT TTC TAC TTC AAG CTG TTC AAA AAC TTC AAG
     GTC AGA GTC TAG CAA AGA AAG ATG AAG TTC GAC AAG TTT TTG AAG TTC

                            70                                           80
     GAC GAC CAG TCT ATC CAG AAA TCT GTT GAA ACT ATC AAG GAA GAC ATG
     CTG CTG GTC AGA TAG GTC TTT AGA CAA CTT TGA TAG TTC CTT CTG TAC

                                        90
     AAC GTT AAG TTC TTC AAC TCT AAC AAG AAA AAG CGT GAC GAC TTC GAA
     TTG CAA TTC AAG AAG TTG AGA TTG TTC TTT TTC GCA CTG CTG AAG CTT

                    100                                          110
     AAG CTT ACT AAC TAC TCT GTT ACT GAC CTT AAT GTA CAG CGT AAA GCT
     TTC GAA TGA TTG ATG AGA CAA TGA CTG GAA TTA CAT GTC GCA TTT CGA

                                        120
     ATC CAT GAA CTG ATC CAG GTT ATG GCT GAA CTG TCC CCG GCT GCT AAA
     TAG GTA CTT GAC TAG GTC CAA TAC CGA CTT GAC AGG GGC CGA CGA TTT

            130                                          140
     ACT GGT AAG CGT AAA AGA TCT CAG ATG CTG TTC CGT GGT CGT CGT GCT
     TGA CCA TTC GCA TTT TCT AGA GTC TAC GAC AAG GCA CCA GCA GCA CGA

            146
     TCT CAG
     AGA GTC
```

dans laquelle $\begin{smallmatrix}1\\ \text{TGC}\\ \text{ACG}\end{smallmatrix}$ peut être remplacé par $\begin{smallmatrix}1\\ \text{TGT}\\ \text{ACA}\end{smallmatrix}$

codant pour la séquence d'acides aminés

```
        1                                    10
        Cys Tyr Cys Gln Asp Pro Tyr Val Lys Glu Ala Glu
                                20
        Asn Leu Lys Lys Tyr Phe Asn Ala Gly His Ser Asp Val
                    30
        Ala Asp Asn Gly Thr Leu Phe Leu Gly Ile Leu Lys Asn
            40                                          50
        Trp Lys Glu Glu Ser Asp Arg Lys Ile Met Gln Ser Gln
                                        60
        Ile Val Ser Phe Tyr Phe Lys Leu Phe Lys Asn Phe Lys
                            70
        Asp Asp Gln Ser Ile Gln Lys Ser Val Glu Thr Ile Lys
                80                                      90
        Glu Asp Met Asn Val Lys Phe Phe Asn Ser Asn Lys Lys
                                        100
        Lys Arg Asp Asp Phe Glu Lys Leu Thr Asn Tyr Ser Val
                                110
        Thr Asp Leu Asn Val Gln Arg Lys Ala Ile His Glu Leu
                    120
        Ile Gln Val Met Ala Glu Leu Ser Pro Ala Ala Lys Thr
        130                                     140
        Gly Lys Arg Lys Arg Ser Gln Met Leu Phe Arg Gly Arg

        Arg Ala Ser Gln
```

et une séquence de nucléotides suivante codant pour l'arrêt de la traduction, qui y est insérée, en provoquant ainsi la formation du polypeptide ayant la séquence d'acides aminés précédente comme composant protéique dans le bouillon de culture.

2. Procédé selon la revendication 1, dans lequel les cellules hôtes sont d'une souche appartenant au genre *Escherichia*.

3. Procédé selon la revendication 2, dans lequel les cellules hôtes sont d'une souche appartenant à l'espèce *Escherichia coli.*

4. Procédé selon la revendication 3, dans lequel les cellules hôtes transformées sont de la souche *Escherichia coli* K12.WA802.

5. Procédé selon l'une quelconque des revendications précédentes, dans lequel le polypeptide similaire à l'interféron $\gamma$ est récupéré dans le bouillon de culture.

6. Polydésoxyribonucléotide ayant la séquence

```
        1                                          10
       TGC TAC TGC CAG GAC CCA TAC GTG AAG GAA GCT GAA
       ACG ATG ACG GTC CTG GGT ATG CAC TTC CTT CGA CTT


                                   20
       AAC CTG AAG AAA TAC TTC AAC GCT GGT CAT TCT GAC GTT
       TTG GAC TTC TTT ATG AAG TTG CGA CCA GTA AGA CTG CAA


                       30
       GCT GAC AAC GGT ACT CTG TTC CTG GGT ATC CTG AAA AAC
       CGA CTG TTG CCA TGA GAC AAG GAC CCA TAG GAC TTT TTG


               40                                  50
       TGG AAA GAA GAA TCT GAC CGT AAA ATC ATG CAG TCT CAG
       ACC TTT CTT CTT AGA CTG GCA TTT TAG TAC GTC AGA GTC


                                   60
       ATC GTT TCT TTC TAC TTC AAG CTG TTC AAA AAC TTC AAG
       TAG CAA AGA AAG ATG AAG TTC GAC AAG TTT TTG AAG TTC


                           70
       GAC GAC CAG TCT ATC CAG AAA TCT GTT GAA ACT ATC AAG
       CTG CTG GTC AGA TAG GTC TTT AGA CAA CTT TGA TAG TTC


           80                                      90
       GAA GAC ATG AAC GTT AAG TTC TTC AAC TCT AAC AAG AAA
       CTT CTG TAC TTG CAA TTC AAG AAG TTG AGA TTG TTC TTT


                                       100
       AAG CGT GAC GAC TTC GAA AAG CTT ACT AAC TAC TCT GTT
       TTC GCA CTG CTG AAG CTT TTC GAA TGA TTG ATG AGA CAA


                               110
       ACT GAC CTT AAT GTA CAG CGT AAA GCT ATC CAT GAA CTG
       TGA CTG GAA TTA CAT GTC GCA TTT CGA TAG GTA CTT GAC


                   120
       ATC CAG GTT ATG GCT GAA CTG TCC CCG GCT GCT AAA ACT
       TAG GTC CAA TAC CGA CTT GAC AGG GGC CGA CGA TTT TGA


       130                                    140
       GGT AAG CGT AAA AGA TCT CAG ATG CTG TTC CGT GGT CGT
       CCA TTC GCA TTT TCT AGA GTC TAC GAC AAG GCA CCA GCA


               146
       CGT GCT TCT CAG
       GCA CGA AGA GTC



                           1                          1
                          TGC                        TGT
       dans laquelle      ACG      peut être remplacé par    ACA
```

7. Polydésoxyribonucléotide selon la revendication 6, ayant en plus de ladite séquence un codon d'initiation de la traduction à l'extrémité 5' et un codon d'arrêt de la traduction à l'extrémité 3'.

8. Polydésoxyribonucléotide selon la revendication 7, ayant des séquences de nucléotides clivables par des enzymes de restriction, ajoutées aux deux extrémités.

9. Polydésoxyribonucléotide selon la revendication 8, dans lequel le côté extrémité 5' est clivable par *EcoRI* et le côté extrémité 3' est clivable par Sall.

10. Plasmide dans lequel est inséré un polydésoxyribonucléotide tel que défini dans l'une quelconque des revendications 6 à 9.

11. Souche appartenant à *Escherichia coli* et transformée par un plasmide selon la revendication 10.

Revendications pour l'Etat contractant AT

1. Procédé de production d'un polypeptide similaire à l'interféron $\gamma$ humain, qui consiste à cultiver dans un milieu nutritif des cellules hôtes transformées par un plasmide ayant un polydésoxyribonucléotide qui a un codon d'initiation de la traduction à l'extrémité 5' suivi d'une séquence de nucléotides consistant en

```
1                                             10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

                20                                30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                                40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

        50                                        60
CAG TCT CAG ATC GTT TCT TTC TAC TTC AAG CTG TTC AAA AAC TTC AAG
GTC AGA GTC TAG CAA AGA AAG ATG AAG TTC GAC AAG TTT TTG AAG TTC

                        70                                80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                                90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT

                100                               110
AAG CTT ACT AAC TAC TCT GTT ACT GAC CTT AAT GTA CAG CGT AAA GCT
TTC GAA TGA TTG ATG AGA CAA TGA CTG GAA TTA CAT GTC GCA TTT CGA

                        120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT

        130                               140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA

        146
TCT CAG
AGA GTC
```

dans laquelle $\begin{matrix}1\\ \text{TGC}\\ \text{ACG}\end{matrix}$ peut être remplacé par $\begin{matrix}1\\ \text{TGT}\\ \text{ACA}\end{matrix}$

codant pour la séquence d'acides aminés

```
        1                                          10
     Cys Tyr Cys Gln Asp Pro Tyr Val Lys Glu Ala Glu
                              20
     Asn Leu Lys Lys Tyr Phe Asn Ala Gly His Ser Asp Val
                      30
     Ala Asp Asn Gly Thr Leu Phe Leu Gly Ile Leu Lys Asn
          40                                      50
     Trp Lys Glu Glu Ser Asp Arg Lys Ile Met Gln Ser Gln
                                      60
     Ile Val Ser Phe Tyr Phe Lys Leu Phe Lys Asn Phe Lys
                          70
     Asp Asp Gln Ser Ile Gln Lys Ser Val Glu Thr Ile Lys
              80                                      90
     Glu Asp Met Asn Val Lys Phe Phe Asn Ser Asn Lys Lys
                                      100
     Lys Arg Asp Asp Phe Glu Lys Leu Thr Asn Tyr Ser Val
                              110
     Thr Asp Leu Asn Val Gln Arg Lys Ala Ile His Glu Leu
                  120
     Ile Gln Val Met Ala Glu Leu Ser Pro Ala Ala Lys Thr
     130                                          140
     Gly Lys Arg Lys Arg Ser Gln Met Leu Phe Arg Gly Arg

     Arg Ala Ser Gln
```

et une séquence de nucléotides suivante codant pour l'arrêt de la traduction, qui y est insérée, en provoquant ainsi la formation du polypeptide ayant la séquence d'acides aminés précédente comme composant protéique dans le bouillon de culture.

2. Procédé selon la revendication 1, dans lequel les cellules hôtes sont d'une souche appartenant au genre *Escherichia*.

3. Procédé selon la revendication 2, dans lequel les cellules hôtes sont d'une souche appartenant a l'espèce *Escherichia coli*.

4. Procédé selon la revendication 3, dans lequel les cellules hôtes transformées sont de la souche *Escherichia coli* K12.WA802.

5. Procédé selon l'une quelconque des revendications précédentes, dans lequel le polypeptide similaire à l'interféron $\gamma$ est récupéré dans le bouillon de culture.

6. Procédé selon l'une quelconque des revendications précédentes, dans lequel ladite séquence de nucléotides comporte, ajoutées aux deux extrémités, des séquences de nucléotides clivables par des enzymes de restriction.

7. Procédé selon la revendication 6, dans lequel le côté extrémité 5' est clivable par *EcoRI* et le côté extrémité 3' est clivable par *Sall*.

**Ansprüche**

Patentansprüche für die Vestragsstaaten: BE, CH, DE, FR, GB, IT, LI, LU, NL, SE

1. Verfahren zur Herstellung eines dem menschlichen Gamma-Interferon ähnlichen Polypeptids, umfassend das Kultivieren in einem Nährmedium von Wirtszellen, die mit einem Plasmid transformiert sind, welches ein Polydesoxyribonukleotid aufweist, das am 5'-Ende ein Translations-Initiations-Codon aufweist, gefolgt von einer Nukleotidsequenz bestehend aus

```
1                                   10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

          20                                  30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                              40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

      50                                  60
CAG TCT CAG ATC GTT TCT TTC TAC TTC AAG CTG TTC AAA AAC TTC AAG
GTC AGA GTC TAG CAA ACA AAG ATG AAG TTC GAC AAG TTT TTG AAG TTC

                          70                              80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                              90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT

          100                                 110
AAG CTT ACT AAC TAC TCT GTT ACT GAC CTT ATT GTA CAG CGT AAA GCT
TTC GAA TGA TTG ATG AGA CAA TGA CTG GAA TTA CAT GTC GCA TTT CGA

                              120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT

      130                                 140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA

      146
TCT CAG
AGA GTC
```

1 1

in der TGC ersetzt werden kann durch TGT,

ACG ACA

kodierend für die Aminosäurensequenz

1 10
Cys Tyr Cys Gln Asp Pro Tyr Val Lys Glu Ala Glu

20
Asn Leu Lys Lys Tyr Phe Asn Ala Gly His Ser Asp Val

30
Ala Asp Asn Gly Thr Leu Phe Leu Gly Ile Leu Lys Asn

40 50
Trp Lys Glu Glu Ser Asp Arg Lys Ile Met Gln Ser Gln

60
Ile Val Ser Phe Tyr Phe Lys Leu Phe Lys Asn Phe Lys

70
Asp Asp Gln Ser Ile Gln Lys Ser Val Glu Thr Ile Lys

80 90
Glu Asp Met Asn Val Lys Phe Phe Asn Ser Asn Lys Lys

100
Lys Arg Asp Asp Phe Glu Lys Leu Thr Asn Tyr Ser Val

110
Thr Asp Leu Asn Val Gln Arg Lys Ala Ile His Glu Leu

120
Ile Gln Val Met Ala Glu Leu Ser Pro Ala Ala Lys Thr

130 140
Gly Lys Arg Lys Arg Ser Gln Met Leu Phe Arg Gly Arg

Arg Ala Ser Gln

und einer nachfolgenden, darin eingebauten Nukleotidsequenz, die für die Termination der Translation kodiert und dadurch die Bildung des Polypeptids mit der oben genannten Aminosäuresequenz als eine Proteinkomponente in der Kulturbrühe verursacht.

2.  Verfahren nach Anspruch 1, bei dem die Wirtszellen von einem zur Gattung Escherichia gehörenden Stamm sind.

3.  Verfahren nach Anspruch 2, bei dem die Wirtszellen von einem zur Species Escherichia coli gehörenden Stamm sind.

4.  Verfahren nach Anspruch 3, bei dem die transformierten Wirtszellen Escherichia coli K12.WA802 sind.

5.  Verfahren nach einem der vorhergehenden Ansprüche, bei dem das dem Gamma-Interferon ähnliche Polypeptid aus der Kulturbrühe gewonnen wird.

6.  Polydesoxyribunokleotid der Sequenz

EP 0 095 350 B1

```
      1                                            10
     TGC TAC TGC CAG GAC CCA TAC GTG AAG GAA GCT GAA
     ACG ATG ACG GTC CTG GGT ATG CAC TTC CTT CGA CTT


                                  20
    AAC CTG AAG AAA TAC TTC AAC GCT GGT CAT TCT GAC GTT
    TTG GAC TTC TTT ATG AAG TTG CGA CCA GTA AGA CTG CAA


                      30
   GCT GAC AAC GGT ACT CTG TTC CTG GGT ATC CTG AAA AAC
   CGA CTG TTG CCA TGA GAC AAG GAC CCA TAG GAC TTT TTG


              40                                50
  TGG AAA GAA GAA TCT GAC CGT AAA ATC ATG CAG TCT CAG
  ACC TTT CTT CTT AGA CTG GCA TTT TAG TAC GTC AGA GTC


                              60
 ATC GTT TCT TTC TAC TTC AAG CTG TTC AAA AAC TTC AAG
 TAG CAA AGA AAG ATG AAG TTC GAC AAG TTT TTG AAG TTC


                      70
 GAC GAC CAG TCT ATC CAG AAA TCT GTT GAA ACT ATC AAG
 CTG CTG GTC AGA TAG GTC TTT AGA CAA CTT TGA TAG TTC


              80                                90
 GAA GAC ATG AAC GTT AAG TTC TTC AAC TCT AAC AAG AAA
 CTT CTG TAC TTG CAA TTC AAG AAG TTG AGA TTG TTC TTT


                                    100
 AAG CGT GAC GAC TTC GAA AAG CTT ACT AAC TAC TCT GTT
 TTC GCA CTG CTG AAG CTT TTC GAA TGA TTG ATG AGA CAA


                      110
 ACT GAC CTT AAT GTA CAG CGT AAA GCT ATC CAT GAA CTG
 TGA CTG GAA TTA CAT GTC GCA TTT CGA TAG GTA CTT GAC


              120
 ATC CAG GTT ATG GCT GAA CTG TCC CCG GCT GCT AAA ACT
 TAG GTC CAA TAC CGA CTT GAC AGG GGC CGA CGA TTT TGA


  130                                    140
 GGT AAG CGT AAA AGA TCT CAG ATG CTG TTC CGT GGT CGT
 CCA TTC GCA TTT TCT AGA GTC TAC GAC AAG GCA CCA GCA


          146
 CGT GCT TCT CAG
 GCA CGA AGA GTC ,
```

28

$$\text{in der} \quad \overset{1}{\underset{\text{ACG}}{\text{TGC}}} \quad \text{ersetzt werden kann durch} \quad \overset{1}{\underset{\text{ACA}}{\text{TGT}}}.$$

7. Polydesoxyribonukleotid nach Anspruch 6, welches zusätzlich zur Sequenz ein Translations-Initiations-Codon am 5'-Ende und ein Translations-Terminations-Codon am 3'-Ende aufweist.

8. Polydesoxyribonukleotid nach Anspruch 7, welches mit Restriktionsenzymen spaltbare Nukleotidsequenzen an beiden Enden angehängt aufweist.

9. Polydesoxyribonukleotid nach Anspruch 8, bei dem sich die Seite des 5'-Endes mit Eco RI und die Seite des 3'-Endes mit Sal I schneiden läßt.

10. Plasmid mit einem darin eingebauten Polydesoxyribonukleotid, wie es in einem der Ansprüche 6 bis 9 definiert ist.

11. Zu Escherichia coli gehörender Stamm, der mit einem Plasmid nach Anspruch 10 transformiert ist.

Patent ansprüche für den Vertrapsstaat: AT

1. Verfahren zur Herstellung eines dem menschlichen Gamma-Interferon ähnlichen Polypeptids, umfassend das Kultivieren in einem Nährmedium von Wirtszellen, die mit einem Plasmid transformiert sind, welches ein Polydesoxyribonukleotid aufweist, das am 5'-Ende ein Translations-Initiations-Codon trägt, gefolgt von einer Nukleotidsequenz bestehend aus:

```
1                                          10
TGC TAC TGC CAG GAC CCA TAC GTG AAG GAA GCT GAA AAC CTG AAG AAA
ACG ATG ACG GTC CTG GGT ATG CAC TTC CTT CGA CTT TTG GAC TTC TTT

                20                                          30
TAC TTC AAC GCT GGT CAT TCT GAC GTT GCT GAC AAC GGT ACT CTG TTC
ATG AAG TTG CGA CCA GTA AGA CTG CAA CGA CTG TTG CCA TGA GAC AAG

                                        40
CTG GGT ATC CTG AAA AAC TGG AAA GAA GAA TCT GAC CGT AAA ATC ATG
GAC CCA TAG GAC TTT TTG ACC TTT CTT CTT AGA CTG GCA TTT TAG TAC

        50                                          60
CAG TCT CAG ATC GTT TCT TTC TAC TTC AAG CTG TTC AAA AAC TTC AAG
GTC AGA GTC TAG CAA ACA AAG ATG AAG TTC GAC AAG TTT TTG AAG TTC

                        70                                          80
GAC GAC CAG TCT ATC CAG AAA TCT GTT GAA ACT ATC AAG GAA GAC ATG
CTG CTG GTC AGA TAG GTC TTT AGA CAA CTT TGA TAG TTC CTT CTG TAC

                                        90
AAC GTT AAG TTC TTC AAC TCT AAC AAG AAA AAG CGT GAC GAC TTC GAA
TTG CAA TTC AAG AAG TTG AGA TTG TTC TTT TTC GCA CTG CTG AAG CTT

        100                                         110
AAG CTT ACT AAC TAC TCT GTT ACT GAC CTT ATT GTA CAG CGT AAA GCT
TTC GAA TGA TTG ATG AGA CAA TGA CTG GAA TTA CAT GTC GCA TTT CGA

                                120
ATC CAT GAA CTG ATC CAG GTT ATG GCT GAA CTG TCC CCG GCT GCT AAA
TAG GTA CTT GAC TAG GTC CAA TAC CGA CTT GAC AGG GGC CGA CGA TTT

        130                                         140
ACT GGT AAG CGT AAA AGA TCT CAG ATG CTG TTC CGT GGT CGT CGT GCT
TGA CCA TTC GCA TTT TCT AGA GTC TAC GAC AAG GCA CCA GCA GCA CGA

        146
TCT CAG
AGA GTC
```

in der TGC ersetzt werden kann durch TGT
ACG                                  ACA,

kodierend für die Aminosäuresequenz

```
        1                                                  10
        Cys Tyr Cys Gln Asp Pro Tyr Val Lys Glu Ala Glu
                                    20
    Asn Leu Lys Lys Tyr Phe Asn Ala Gly His Ser Asp Val
                    30
    Ala Asp Asn Gly Thr Leu Phe Leu Gly Ile Leu Lys Asn
        40                                              50
    Trp Lys Glu Glu Ser Asp Arg Lys Ile Met Gln Ser Gln
                                    60
    Ile Val Ser Phe Tyr Phe Lys Leu Phe Lys Asn Phe Lys
                    70
    Asp Asp Gln Ser Ile Gln Lys Ser Val Glu Thr Ile Lys
            80                                          90
    Glu Asp Met Asn Val Lys Phe Phe Asn Ser Asn Lys Lys
                                100
    Lys Arg Asp Asp Phe Glu Lys Leu Thr Asn Tyr Ser Val
                        110
    Thr Asp Leu Asn Val Gln Arg Lys Ala Ile His Glu Leu
                120
    Ile Gln Val Met Ala Glu Leu Ser Pro Ala Ala Lys Thr
    130                                     140
    Gly Lys Arg Lys Arg Ser Gln Met Leu Phe Arg Gly Arg

    Arg Ala Ser Gln ,
```

und einer nachfolgenden, darin eingebauten Nukleotidsequenz, die für die Termination der Translation codiert und dadurch die Bildung des Polypeptids mit der oben genannten Aminosäuresequenz als eine Proteinkomponente in der Kulturbrühe verursacht.

2. Verfahren nach Anspruch 1, bei dem die Wirtszellen von einem zur Gattung Escherichia gehörenden Stamm sind.

3. Verfahren nach Anspruch 2, bei dem die Wirtszellen von einem der Spezies Escherichia coli gehörenden Stamm sind.

4. Verfahren nach Anspruch 3, bei dem die transformierten Wirtszellen Escherichia coli K12.WA802 sind.

5. Verfahren nach einem der vorhergehenden Ansprüche, bei dem das dem Gamma-Inferon ähnliche Polypeptid aus der Kulturbrühe gewonnen wird.

6. Verfahren nach einem der vorhergehenden Ansprüche, bei dem die Nukleotidsequenz mit Restriktionsenzymen spaltbare Nukleotidsequenzen an beiden Enden angehängt aufweist.

7. Verfahren nach Anspruch 6, bei dem sich die Seite des 5'-Endes mit Eco RI und die Seite des 3'-Endes mit Sal I schneiden läßt.

# Fig.1-1

1) AATTCATGTGC
2) GGCAGTAGCACATG
3) TACTGCCAGGACCC
4) CACGTATGGGTCCT
5) ATACGTGAAGGAAG
6) TTTTCAGCTTCCTT
7) CTGAAAACCTGAAG
8) AAGTATTTCTTCAGG
9) AAATACTTCAACGCT
10) AATGACCAGCGTTG

11) GGTCATTCTGACGT
12) GTCAGCAACGTCAG
13) TGCTGAGAACGGTAC
14) GAACAGAGTACCGTT
15) TCTGTTCCTGGGT
16) TCAGGATACCCAG
17) ATCCTGAAAAACTG
18) TTCTTTCCAGTTTT
19) GAAAGAAGAATCTG
20) TTACGGTCAGATTC
21) ACCGTAAAATCATG

22) GAGACTGCATGATT
23) CAGTCTCAGATCGTT
24) AGAAAGAAACGATCT
25) TCTTTCTACTTCAAG
26) TGAACAGCTTGAAGT
27) CTGTTCAAAAACTT
28) GTCCTTGAAGTTTT
29) CAAGGACGACCAGT
30) TGGATAGACTGGTC
31) CTATCCAGAAATCT
32) TTTCAACAGATTTC

33) GTTGAAACTATCAAG
34) ATGTCTTCCTTGATAG
35) GAAGACATGAACGTT
36) AAGAACTTAACGTTC
37) AAGTTCTTCAACTCT
38) TTCTTGTTAGAGTTG
39) AACAAGAAAAGCGT
40) AAGTCGTCACGCTTT
41) GACGACTTCGAAAAGC
42) TTAGTAAGCTTTTCG

43) TTACTAACTACTCT
44) CAGTAACAGAGTAG
45) GTTACTGACCTTAAT
46) GCTGTACATTAAGGT
47) GTACAGCGTAAAGC
48) ATGGATAGCTTTAC
49) TATCCATGAACTGAT
50) AACCTGGATCAGTTC
51) CCAGGTTATGGCTG
52) GGACAGTTCAGCCAT

53) AACTGTCCCCGGCTGC
54) CAGTTTTAGCAGCCGG
55) TAAAACTGGTAAGCGT
56) ATCTTTTACGCTTAC
57) AAAAGATCTCAGATG
58) GGAACAGCATCTGAG
59) CTGTTCCGTGGTCGT
60) AAGCACGACGACCAC
61) CGTGCTTCTCAGTAAG
62) TCGACTTACTGAG

EP 0 095 350 B1

## Fig. 1-2

```
              1                                        10                                      20
              Met Cys Tyr Cys Gln Asp Pro Tyr Val Lys Glu Ala Glu Asn Leu Lys Lys Tyr Phe Asn Ala Gly His Ser Asp
5'AATTC ATG  TGC TAC TGC CAG GAC CCA TAC GTG AAG GAA GCT GAA AAC CTG AAG AAA TAC TTC AAC GCT GGT CAT TCT GAC
  3'  G TAC ACG ATG ACG GTC CTG GGT ATG CAC TTC CTT CGA CTT TTG GAC TTC TTT ATG AAG TTG CGA CCA GTA AGA CTG

                              30                                      40
              Val Ala Asp Asn Gly Thr Leu Phe Leu Gly Ile Leu Lys Asn Trp Lys Glu Glu Ser Asp Arg Lys Ile Met Gln
              GTT GCT GAC AAC GGT ACT CTG TTC CTG GGT ATC CTG AAA AAC TGG AAA GAA GAA TCT GAC CGT AAA ATC ATG CAG
              CAA CGA CTG TTG CCA TGA GAC AAG GAC CCA TAG GAC TTT TTG ACC TTT CTT CTT AGA CTG GCA TTT TAG TAC GTC

              50                                      60                                      70
              Ser Gln Ile Val Ser Phe Tyr Phe Lys Leu Phe Lys Asn Phe Lys Asp Asp Gln Ser Ile Gln Lys Ser Val Glu
              TCT CAG ATC GTT TCT TTC TAC TTC AAG CTG TTC AAA AAC TTC AAG GAC GAC CAG TCT ATC CAG AAA TCT GTT GAA
              AGA GTC TAG CAA AGA AAG ATG AAG TTC GAC AAG TTT TTG AAG TTC CTG CTG GTC AGA TAG GTC TTT AGA CAA CTT

                              80                                      90
              Thr Ile Lys Glu Asp Met Asn Val Lys Phe Phe Asn Ser Asn Lys Lys Lys Arg Asp Asp Phe Glu Lys Leu Thr
              ACT ATC AAG GAA GAC ATG AAC GTT AAG TTC TTC AAC TCT AAC AAG AAA AAG CGT GAC GAC TTC GAA AAG CTT ACT
              TGA TAG TTC CTT CTG TAC TTG CAA TTC AAG AAG TTG AGA TTG TTC TTT TTC GCA CTG CTG AAG CTT TTC GAA TGA

              100                                     110                                     120
              Asn Tyr Ser Val Thr Asp Leu Asn Val Gln Arg Lys Ala Ile His Glu Leu Ile Gln Val Met Ala Glu Leu Ser
              AAC TAC TCT GTT ACT GAC CTT AAT GTA CAG CGT AAA GCT ATC CAT GAA CTG ATC CAG GTT ATG GCT GAA CTG TCC
              TTG ATG AGA CAA TGA CTG GAA TTA CAT GTC GCA TTT CGA TAG GTA CTT GAC TAG GTC CAA TAC CGA CTT GAC AGG

                              130                                     140                     146
              Pro Ala Ala Lys Thr Gly Lys Arg Lys Arg Ser Gln Met Leu Phe Arg Gly Arg Arg Ala Ser Gln Stp
              CCG GCT GCT AAA ACT GGT AAG CGT AAA AGA TCT CAG ATG CTG TTC CGT GGT CGT CGT GCT TCT CAG TAA G  3'
              GGC CGA CGA TTT TGA CCA TTC GCA TTT TCT AGA GTC TAC GAC AAG GCA CCA GCA GCA CGA AGA GTC ATT CAGCT 5'
```

EP 0 095 350 B1

# Fig. 2

EP 0 095 350 B1

EP 0 095 350 B1

# Fig. 3

$$n = 0 \text{ or } 1$$

Fig. 4

EP 0 095 350 B1

# Fig. 5

EcoRI (1) (G↓AATTC)

AvaⅡ (21) (G↓G$\frac{A}{T}$CC)

Fig. 6

— 100

HinfI (133) (G↓ANTC)

DdeI (158) (C↓TNAG)

— 200

TaqI (293) (T↓CGA)

HindⅢ (298) (A↓AGCTT)

— 400

BglⅡ (409) (A↓GATCT)
DdeI (413)

DdeI (443)
SalI (451)   TaqI (452)
(G↓TCGAC)

# Fig. 7

LacUV5 promoter

EcoRI (4459)

Hind III ( 6 )

BamHI (352)

SalI ( 627 )

Pst I (3585)

Amp$^r$

Tet$^r$

pK0703

Amp$^r$, Tet$^r$

( 4459 base pairs)

Ava I ( 1401 )

# Fig. 8

i) EcoRI + ClaI digestion
ii) Select large fragment
iii) T4 DNA polymerase

—GAATT    CGAT—
—CTTAA    GCTA—

Lacuv5PO

EcoRI
ClaI

BamHI

Ap$^r$    Tc$^r$    ←SalI

pBR322

T4DNA ligase

EcoRI

Ap$^r$    Tc$^r$    ←SalI

pKO703

UV5PO

Lac

AATTCT————————AGAATT
TTAAGA————————TCTTAA

i) EcoRI digestion fragment
ii) Select digested fragment
iii) T4DNA polymerase

i) EcoRI + SalI digestion
ii) Select large fragment

pKO601

———— Chemically synthesized
α-IFN gene

T4 DNA ligase

EcoRI

LacPO→        γ-IFN

Ap$^r$
pGIF4        ←SalI

40

Fig.9

← 200

← 151
← 140

← 118

← 100

← 82

← 66

Fig. 10

← 311

← 249

← 200

← 151
← 140

← 118

← 100

← 82

← 66

← 48

FIG.11

FIG. 12

44